# EUROPÄISCHE PATENTSCHRIFT

(11) **EP 1 765 824 B1**
(45) Veröffentlichungstag und Bekanntmachung des Hinweises auf die Patenterteilung: **09.01.2008**
(21) Anmeldenummer: 05762108.8
(22) Anmeldetag: 06.07.2005
(51) Int. Cl.: C07D 487/04, A01N 43/90

(54) **SUBSTITUIERTE 6-PHENYL-7-AMINO-TRIAZOLOPYRIMIDINE, VERFAHREN ZU IHRER HERSTELLUNG UND IHRE VERWENDUNG ZUR BEKÄMPFUNG VON SCHADPILZEN SOWIE SIE ENTHALTENDE MITTEL**
SUBSTITUTED 6-PHENYL-7-AMINO-TRIAZOLOPYRIMIDINES, METHOD FOR THE PRODUCTION THEREOF, THEIR USE FOR CONTROLLING PATHOGENIC FUNGI, AND AGENTS CONTAINING THESE COMPOUNDS
6-PHENYL-7-AMINO-TRIAZOLOPYRIMIDINES SUBSTITUEES, PROCEDES PERMETTANT DE LES PRODUIRE ET LEUR UTILISATION POUR LUTTER CONTRE DES CHAMPIGNONS NUISIBLES, ET AGENTS LES CONTENANT

(30) Priorität: 08.07.2004 DE 102004033239
(43) Veröffentlichungstag der Anmeldung: 28.03.2007
(73) Patentinhaber: BASF AKTIENGESELLSCHAFT, 67056 Ludwigshafen (DE)
(72) Erfinder: BLETTNER, Carsten, 68165 Mannheim (DE); GEWEHR, Markus, 56288 Kastellaun (DE); GRAMMENOS, Wassilios, 67071 Ludwigshafen (DE); GROTE, Thomas, 67157 Wachenheim (DE); HÜNGER, Udo, 55124 Mainz (DE); MÜLLER, Bernd, 67227 Frankenthal (DE); NIEDENBRÜCK, Matthias, 67117 Limburgerhof (DE); RHEINHEIMER, Joachim, 67063 Ludwigshafen (DE); SCHÄFER, Peter, 67308 Ottersheim (DE); SCHIEWECK, Frank, 67258 Hessheim (DE); SCHWÖGLER, Anja, 68165 Mannheim (DE); WAGNER, Oliver, 67433 Neustadt (DE); NAVE, Barbara, 67146 Deidesheim (DE); SCHERER, Maria, 76829 Landau-Godramstein (DE); STRATHMANN, Siegfried, 67117 Limburgerhof (DE); SCHÖFL, Ulrich, 68782 Brühl (DE); STIERL, Reinhard, 67251 Freinsheim (DE)
(86) Internationale Anmeldenummer: PCT/EP2005/007277
(87) Internationale Veröffentlichungsnummer: WO 2006/005492

(56) Entgegenhaltungen:
- EP-A- 0 550 113
- WO-A-03/008416
- JP-A- 2002 308 879
- US-A- 4 567 263
- US-A- 5 994 360

## Beschreibung

Die vorliegende Erfindung betrifft substituierte Triazolopyrimidine der Formel I 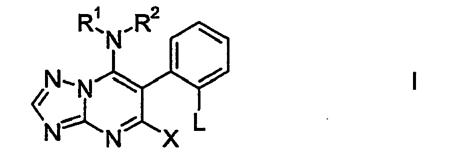 in der die Substituenten folgende Bedeutung haben:
- R¹: C₁-C₈-Alkyl, C₁-C₈-Halogenalkyl, C₃-C₈-Cycloalkyl, C₃-C₈-Halogencycloalkyl, C₂-C₈-Alkenyl, C₂-C₈-Halogenalkenyl, C₃-C₆-Cycloalkenyl, C₃-C₆-Halogencycloalkenyl, C₂-C₈-Alkinyl, C₂-C₈-Halogenalkinyl oder Phenyl, Naphthyl, oder ein fünf- oder sechsgliedriger gesättigter, partiell ungesättigter oder aromatischer Heterocyclus, enthaltend ein bis vier Heteroatome aus der Gruppe O, N oder S,
- R²: Wasserstoff oder eine der bei R¹ genannten Gruppen,
R¹ und R² können auch zusammen mit dem Stickstoffatom, an das sie gebunden sind, ein fünf- oder sechsgliedriges Heterocyclyl oder Heteroaryl bilden, welches über N gebunden ist und ein bis drei weitere Heteroatome aus der Gruppe O, N und S als Ringglied enthalten und/oder einen oder mehrere Substituenten aus der Gruppe Halogen, C₁-C₆-Alkyl, C₁-C₆-Halogenalkyl, C₂-C₆-Alkenyl, C₂-C₆-Halogenalkenyl, C₁-C₆-Alkoxy, C₁-C₆-Halogenalkoxy, C₃-C₆-Alkenyloxy, C₃-C₆-Halogenalkenyloxy, (exo)-C₁-C₆-Alkylen und Oxy-C₁-C₃-alkylenoxy tragen kann;
R¹ und/oder R² können eine bis vier gleiche oder verschiedene Gruppen R^{a} tragen:
- R^{a}: Halogen, Cyano, Nitro, Hydroxy, C₁-C₆-Alkyl, C₁-C₆-Halogenalkyl, C₁-C₆-Alkylcarbonyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₈-Alkenyl, C₂-C₈-Halogenalkenyl, C₃-C₈-Cycloalkenyl, C₂-C₆-Alkenyloxy, C₃-C₆-Halogenalkenyloxy, C₂-C₆-Alkinyl, C₂-C₆-Halogenalkinyl, C₃-C₆-Alkinyloxy, C₃-C₆-Halogenalkinyloxy, C₃-C₆-Cycloalkoxy, C₃-C₆-Cycloalkenyloxy, Oxy-C₁-C₃-alkylenoxy, Phenyl, Naphthyl, fünf- bis zehngliedriger gesättigter, partiell ungesättigter oder aromatischer Heterocyclus, enthaltend ein bis vier Heteroatome aus der Gruppe O, N oder S,
wobei diese aliphatischen, alicyclischen oder aromatischen Gruppen ihrerseits partiell oder vollständig halogeniert sein oder eine bis drei Gruppen R^{b} tragen können:
R^{b} Halogen, Cyano, Nitro, Hydroxy, Mercapto, Amino, Carboxyl, Aminocarbonyl, Aminothiocarbonyl, Alkyl, Haloalkyl, Alkenyl, Alkenyloxy, Alkinyloxy, Alkoxy, Halogenalkoxy, Alkylthio, Alkylamino, Dialkylamino, Formyl, Alkylcarbonyl, Alkylsulfonyl, Alkylsulfoxyl, Alkoxycarbonyl, Alkylcarbonyloxy, Alkylaminocarbonyl, Dialkylaminocarbonyl, Alkylaminothiocarbonyl, Dialkylaminothiocarbonyl, wobei die Alkylgruppen in diesen Resten 1 bis 6 Kohlenstoffatome enthalten und die genannten Alkenyl- oder Alkinylgruppen in diesen Resten 2 bis 8 Kohlenstoff atome enthalten;
und/oder einen bis drei der folgenden Reste:
Cycloalkyl, Cycloalkoxy, Heterocyclyl, Heterocyclyloxy, wobei die cyclischen Systeme 3 bis 10 Ringglieder enthalten; Aryl, Aryloxy, Arylthio, Aryl-C₁-C₆-alkoxy, Aryl-C₁-C₆-alkyl, Hetaryl, Hetaryloxy, Hetarylthio, wobei die Arylreste vorzugsweise 6 bis 10 Ringglieder, die Hetarylreste 5 oder 6 Ringglieder enthalten, wobei die cyclischen Systeme partiell oder vollständig halogeniert oder durch Alkyl- oder Haloalkylgruppen substituiert sein können;
- L: Fluor, Chlor oder Methyl, und
- X: Cyano, C₁-C₄-Alkyl, C₁-C₄-Alkoxy oder C₁-C₂-Halogenalkoxy;
wobei X nicht Methyl bedeutet, wenn R¹ und R² gemeinsam n-Pentylen oder 3-Methyl-n-pentylen und L Fluor bedeutet, oder R¹ und R² gemeinsam 3-Methyl-n-pentylen und L Chlor bedeutet.

Außerdem betrifft die Erfindung Verfahren und Zwischenprodukte zur Herstellung dieser Verbindungen, sie enthaltende Mittel sowie ihre Verwendung zur Bekämpfung von pflanzenpathogenen Schadpilzen.

Aus EP-A 71 792, EP-A 550113 sind 5-Chlor-6-phenyl-7-amino-triazolopyrimidine allgemein bekannt. In US 5 994 360 werden einzelne 5-Methyl-6-phenyl-7-amino-triazolopyrimidine offenbart, in JP-A 2002-308879 werden 5-Halogenalkyl-6-phenyl-7-aminotriazolopyrimidine allgemein vorgeschlagen. Diese Verbindungen sind zur Bekämpfung von Schadpilzen bekannt.

Die erfindungsgemäßen Verbindungen unterscheiden sich von den in den vorgenannten Schriften offenbarten Verbindungen durch die spezifische Kombination der orthosubstituierten 6-Phenylgruppe mit den Substituenten in 5- und 7-Position des Triazolopyrimidin-Grundgerüstes.

Die Wirkung der bekannten Verbindungen ist jedoch in vielen Fällen nicht zufriedenstellend. Davon ausgehend, liegt der vorliegenden Erfindung die Aufgabe zugrunde, Verbindungen mit verbesserter Wirkung und/oder verbreitertem Wirkungsspektrum bereitzustellen.

Demgemäss wurden die eingangs definierten Verbindungen gefunden. Des weiteren wurden Verfahren und Zwischenprodukte zu ihrer Herstellung, sie enthaltende Mittel sowie Verfahren zur Bekämpfung von Schadpilzen unter Verwendung der Verbindungen I gefunden.

Die erfindungsgemäßen Verbindungen können auf verschiedenen Wegen erhalten werden.

Die erfindungsgemäßen Verbindungen können auf verschiedenen Wegen erhalten werden. Vorteilhaft werden sie durch Umsetzung von 5-Aminotriazol der Formel II mit entsprechend substituierten Phenylmalonaten der Formel III, in der R für Alkyl, bevorzugt für C₁-C₆-Alkyl, insbesondere für Methyl oder Ethyl steht, dargestellt.

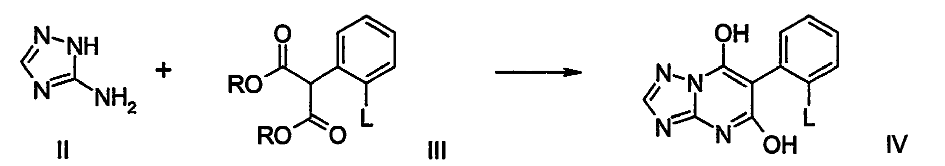

Diese Umsetzung erfolgt üblicherweise bei Temperaturen von 80°C bis 250°C, vorzugsweise 120°C bis 180°C, ohne Solvens oder in einem inerten organischen Lösungsmittel in Gegenwart einer Base [vgl. EP-A 770 615] oder in Gegenwart von Essigsäure unter den aus Adv. Het. Chem. Bd. 57, S. 81ff. (1993) bekannten Bedingungen.

Geeignete Lösungsmittel sind aliphatische Kohlenwasserstoffe, aromatische Kohlenwasserstoffe wie Toluol, o-, m- und p-Xylol, halogenierte Kohlenwasserstoffe, Ether, Nitrile, Ketone, Alkohole, sowie N-Methylpyrrolidon, Dimethylsulfoxid, Dimethylformamid und Dimethylacetamid. Besonders bevorzugt wird die Umsetzung ohne Lösungsmittel oder in Chlorbenzol, Xylol, Dimethylsulfoxid, N-Methylpyrrolidon durchgeführt. Es können auch Gemische der genannten Lösungsmittel verwendet werden.

Als Basen kommen allgemein anorganische Verbindungen wie Alkalimetall- und Erdalkalimetallhydroxide, Alkalimetall- und Erdalkalimetalloxide, Alkalimetall- und Erdalkalimetallhydride, Alkalimetallamide, Alkalimetall- und Erdalkalimetallcarbonate sowie Alkalimetallhydrogencarbonate, metallorganische Verbindungen, insbesondere Alkalimetallalkyle, Alkylmagnesiumhalogenide sowie Alkalimetall- und Erdalkalimetallalkoholate und Dimethoxymagnesium, außerdem organische Basen, z.B. tertiäre Amine wie Trimethylamin, Triethylamin, Tri-isopropylethylamin, Tributylamin und N-Methylpiperidin, N-Methylmorpholin, Pyridin, substituierte Pyridine wie Collidin, Lutidin und 4-Dimethylaminopyridin sowie bicyclische Amine in Betracht. Besonders bevorzugt werden tertiäre Amine wie Tri-isopropylethylamin, Tributylamin, N-Methylmorpholin oder N-Methyl-piperidin.

Die Basen werden im allgemeinen in katalytischen Mengen eingesetzt, sie können aber auch äquimolar, im Überschuss oder gegebenenfalls als Lösungsmittel verwendet werden.

Die Edukte werden im allgemeinen in äquimolaren Mengen miteinander umgesetzt. Es kann für die Ausbeute vorteilhaft sein, die Base und das Malonat III in einem Überschuss bezogen auf das Triazol einzusetzen.

Phenylmalonate der Formel III werden vorteilhaft aus der Reaktion entsprechend substituierter Brombenzole mit Dialkylmalonaten unter Cu(I)-Katalyse erhalten [vgl. Chemistry Letters, S. 367-370, 1981; EP-A 10 02 788].

Die Dihydroxytriazolopyrimidine der Formel IV werden unter den aus WO-A 94/20501 bekannten Bedingungen in die Dihalogenpyrimidine der Formel V überführt, in der Hal ein Halogenatom, bevorzugt ein Brom oder ein Chloratom, insbesondere ein Chloratom bedeutet. Als Halogenierungsmittel [HAL] wird vorteilhaft ein Chlorierungsmittel oder ein Bromierungsmittel, wie Phosphoroxybromid oder Phosphoroxychlorid, ggf. in Anwesenheit eines Lösungsmittels, eingesetzt.

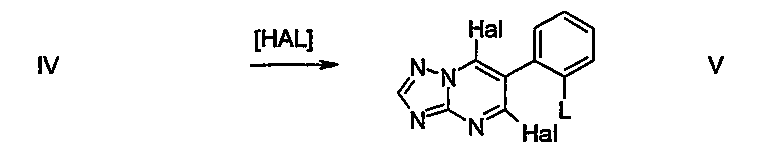

Diese Umsetzung wird üblicherweise bei 0°C bis 150°C, bevorzugt bei 80°C bis 125°C, durchgeführt [vgl. EP-A 770 615].

Dihalogenpyrimidine der Formel V werden mit Aminen der Formel VI, 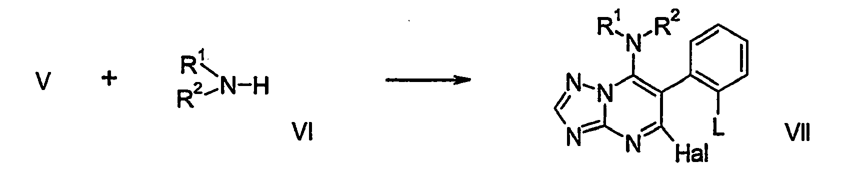 in der R¹ und R² wie in Formel 1 definiert sind, zu 5-Halogen-triazolopyriminen der Formel VII weiter umgesetzt.

Diese Umsetzung wird vorteilhaft bei 0°C bis 70°C, bevorzugt 10°C bis 35°C durchgeführt, vorzugsweise in Anwesenheit eines inerten Lösungsmittels, wie Ether, z. B. Dioxan, Diethylether oder insbesondere Tetrahydrofuran, halogenierte Kohlenwasserstoffe, wie Dichlormethan und aromatische Kohlenwasserstoffe, wie beispielsweise Toluol [vgl. WO-A 98/46608].

Die Verwendung einer Base, wie tertiäre Amine, beispielsweise Triethylamin oder anorganische Amine, wie Kaliumcarbonat ist bevorzugt; auch überschüssiges Amin der Formel VI kann als Base dienen.

Verbindungen der Formel I, in der X Cyano, C₁-C₆-Alkoxy oder C₁-C₂-Halogenalkoxy bedeutet, können vorteilhaft aus der Umsetzung von Verbindungen I, in der X Halogen, bevorzugt Chlor bedeutet, mit Verbindungen M-X' (Formel VIII) erhalten werden. Verbindungen VII stellen je nach der Bedeutung der einzuführenden Gruppe X' ein anorganisches Cyanid, ein Alkoxylat oder ein Halogenalkoxylat dar. Die Umsetzung erfolgt vorteilhaft in Anwesenheit eines inerten Lösungsmittels. Das Kation M in Formel VIII hat geringe Bedeutung; aus praktischen Gründen sind üblicherweise Ammonium-, Tetraalkylammonium- oder Alkali- oder Erdalkalimetallsalze bevorzugt.

I (X = Halogen) + M-X' **→** I (X = X') VIII

Üblicherweise liegt die Reaktionstemperatur bei 0 bis 120°C, bevorzugt bei 10 bis 40°C [vgl. J. Heterocycl. Chem., Bd.12, S. 861-863 (1975)].

Geeignete Lösungsmittel umfassen Ether, wie Dioxan, Diethylether und, bevorzugt Tetrahydrofuran, halogenierte Kohlenwasserstoffe wie Dichlormethan und aromatische Kohlenwasserstoffe, wie Toluol.

Verbindungen der Formel I, in denen X für C₁-C₄-Alkyl steht, können vorteilhaft durch folgenden Syntheseweg erhalten werden:

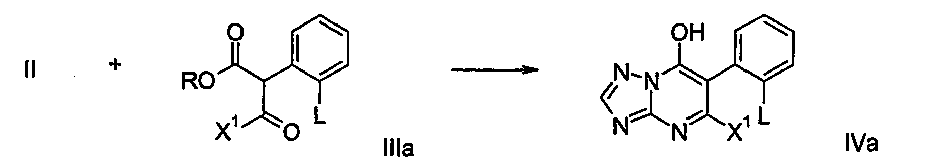

Ausgehend von den Ketoestem IIIa werden die 5-Alkyl-7-hydroxy-6-phenyltriazolopyrimidine IVa erhalten. In Formeln IIIa und IVa steht X¹ für C₁-C₄-Alkyl oder C₁-C₄-Halogenalkyl. Durch Verwendung der leicht zugänglichen 2-Phenylacetessigestem (IIIa mit X¹=CH₃) werden die 5-Methyl-7-hydroxy-6-phenyltriazolopyrimidine erhalten [vgl. Chem. Pharm. Bull., 9, 801, (1961)]. Die Herstellung der Ausgangsverbindungen IIIa erfolgt vorteilhaft unter den aus EP-A 10 02 788 beschrieben Bedingungen.

Die so erhaltenen 5-Alkyl-7-hydroxy-6-phenyltriazolopyrimidine werden mit Halogenierungsmitteln [HAL] unter den weiter oben beschriebenen Bedingungen zu den 7-Halogenotriazolopyrimidinen der Formel Va umgesetzt. Bevorzugt werden Chlorierungs- oder Bromierungsmittel wie Phosphoroxybromid, Phosphoroxychlorid, Thionylchlorid, Thionylbromid oder Sulfurylchlorid eingesetzt. Die Umsetzung kann in Substanz oder in Gegenwart eines Lösungsmittels durchgeführt werden. Übliche Reaktionstemperaturen betragen von 0 bis 150°C oder vorzugsweise von 80 bis 125°C.

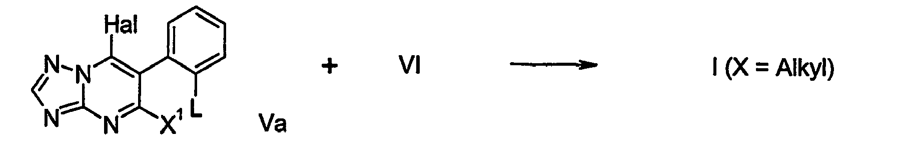

Die Umsetzung von Va mit Aminen VI erfolgt unter den weiter oben beschriebenen Bedingungen.

Verbindungen der Formel I in der X C₁-C₄-Alkyl bedeutet, können alternativ auch aus Verbindungen VII, in der X insbesondere Chlor bedeutet und Malonaten der Formel IX hergestellt werden. In Formel IX bedeuten X" Wasserstoff oder C₁-C₃-Alkyl und R C₁-C₄-Alkyl. Sie werden zu Verbindungen der Formel X umgesetzt und zu Verbindungen I decarboxyliert [vgl. US 5,994,360].

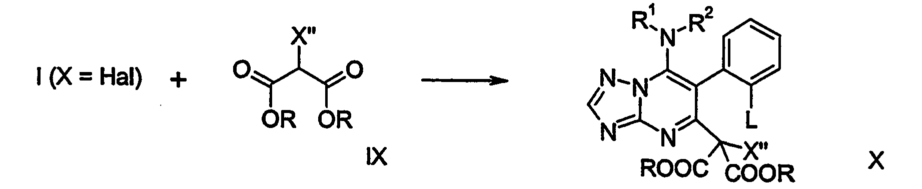

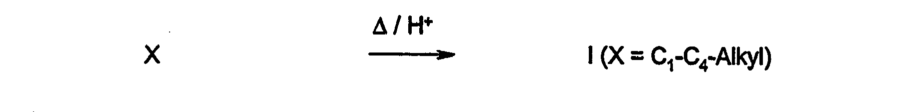

Die Malonate IX sind in der Literatur bekannt [J. Am. Chem. Soc., Bd. 64, 2714 (1942); J. Org. Chem., Bd. 39, 2172 (1974); Helv. Chim. Acta, Bd. 61, 1565 (1978)] oder können gemäß der zitierten Literatur hergestellt werden.

Die anschließende Verseifung des Esters X erfolgt unter allgemein üblichen Bedingungen, in Abhängigkeit der verschiedenen Strukturelemente kann die alkalische oder die saure Verseifung der Verbindungen X vorteilhaft sein. Unter den Bedingungen der Esterverseifung kann die Decarboxylierung zu I bereits ganz oder teilweise erfolgen.

Die Decarboxylierung erfolgt üblicherweise bei Temperaturen von 20°C bis 180°C, vorzugsweise 50°C bis 120°C, in einem inerten Lösungsmittel, gegebenenfalls in Gegenwart einer Säure.

Geeignete Säuren sind Salzsäure, Schwefelsäure, Phosphorsäure, Ameisensäure, Essigsäure, p-Toluolsulfonsäure. Geeignete Lösungsmittel sind Wasser, aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert-Butylmethylketon, Alkohole wie Methanol, Ethanol, n-Propanol, Isopropanol, n-Butanol und tert.-Butanol, sowie Dimethylsulfoxid, Dimethylformamid und Dimethylacetamid, besonders bevorzugt wird die Reaktion in Salzsäure oder Essigsäure durchgeführt. Es können auch Gemische der genannten Lösungsmittel verwendet werden.

Verbindungen der Formel I, in denen X für C₁-C₄-Alkyl steht, können auch durch Kupplung von 5-Halogentriazolopyrimidinen der Formel VII mit metallorganischen Reagenzien der Formel XI erhalten werden. In einer Ausführungsform dieses Verfahrens erfolgt die Umsetzung unter Übergangsmetallkatalyse, wie Ni- oder Pd-Katalyse.

VII + M^{y}(-X")_{y} → I (X = C₁-C₄-Alkyl) XI

In Formel XI steht M für ein Metallion der Wertigkeit Y, wie beispielsweise B, Zn oder Sn und X" für C₁-C₃-Alkyl. Diese Reaktion kann beispielsweise analog folgender Methoden durchgeführt werden: J. Chem. Soc. Perkin Trans. 1, 1187 (1994), ebenda 1, 2345 (1996); WO-A 99/41255; Aust. J. Chem., Bd. 43, 733 (1990); J. Org. Chem., Bd. 43, 358 (1978); J. Chem. Soc. Chem. Commun. 866 (1979); Tetrahedron Lett., Bd. 34, 8267 (1993); ebenda, Bd. 33,413 (1992).

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Sofern einzelne Verbindungen I nicht auf den voranstehend beschriebenen Wegen zugänglich sind, können sie durch Derivatisierung anderer Verbindungen I hergestellt werden.

Sofern bei der Synthese Isomerengemische anfallen, ist im allgemeinen jedoch eine Trennung nicht unbedingt erforderlich, da sich die einzelnen Isomere teilweise während der Aufbereitung für die Anwendung oder bei der Anwendung (z.B. unter Licht-, Säure- oder Baseneinwirkung) ineinander umwandeln können. Entsprechende Umwandlungen können auch nach der Anwendung, beispielsweise bei der Behandlung von Pflanzen in der behandelten Pflanze oder im zu bekämpfenden Schadpilz erfolgen.

Bei den in den vorstehenden Formeln angegebenen Definitionen der Symbole wurden Sammelbegriffe verwendet, die allgemein repräsentativ für die folgenden Substituenten stehen:
Halogen: Fluor, Chlor, Brom und Jod;
Alkyl: gesättigte, geradkettige oder verzweigte Kohlenwasserstoffreste mit 1 bis 4, 6 oder 8 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
Halogenalkyl: geradkettige oder verzweigte Alkylgruppen mit 1 bis 2, 4 oder 6 Kohlenstoffatomen (wie vorstehend genannt), wobei in diesen Gruppen teilweise oder vollständig die Wasserstoffatome durch Halogenatome wie vorstehend genannt ersetzt sein können: insbesondere C₁-C₂Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl oder 1,1,1-Trifluorprop-2-yl; Alkenyl: ungesättigte, geradkettige oder verzweigte Kohlenwasserstoffreste mit 2 bis 4, 6 oder 8 Kohlenstoffatomen und einer oder zwei Doppelbindungen in beliebiger Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1 ,2-Dimethyl-1-propenyl, 1,2-Dimethyl-2-propenyl, 1-Ethyl-1 propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl;
Halogenalkenyl: ungesättigte, geradkettige oder verzweigte Kohlenwasserstoffreste mit 2 bis 8 Kohlenstoffatomen und einer oder zwei Doppelbindungen in beliebiger Position (wie vorstehend genannt), wobei in diesen Gruppen die Wasserstoffatome teilweise oder vollständig gegen Halogenatome wie vorstehend genannt, insbesondere Fluor, Chlor und Brom, ersetzt sein können;
Alkinyl: geradkettige oder verzweigte Kohlenwasserstoffgruppen mit 2 bis 4, 6 oder 8 Kohlenstoffatomen und einer oder zwei Dreifachbindungen in beliebiger Position, z.B. C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-penünyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
Cycloalkyl: mono- oder bicyclische, gesättigte Kohlenwasserstoffgruppen mit 3 bis 6 oder 8 Kohlenstoffringgliedern, z.B. C₃-C₈-Cycloalkyl wie Cyclopropyl, Cyclobutyl, Cyclopentyl, Cyclohexyl, Cycloheptyl und Cyclooctyl;
fünf- bis zehngliedriger gesättigter, partiell ungesättigter oder aromatischer Heterocyclus, enthaltend ein bis vier Heteroatome aus der Gruppe O, N oder S:
   - 5- oder 6-gliedriges Heterocyclyl, enthaltend ein bis drei Stickstoffatome und/oder ein Sauerstoff- oder Schwefelatom oder ein oder zwei Sauerstoff- und/oder Schwefelatome, z.B. 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 2-Pyrrolin-2-yl, 2-Pyrrolin-3-yl, 3-Pyrrolin-2-yl, 3-Pyrrolin-3-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Hexahydropyridazinyl, 4-Hexahydropyridazinyl, 2-Hexahydropyrimidinyl, 4-Hexahydropyrimidinyl, 5-Hexahydropyrimidinyl und 2-Piperazinyl;
   - 5-gliedriges Heteroaryl, enthaltend ein bis vier Stickstoffatome oder ein bis drei Stickstoffatome und ein Schwefel- oder Sauerstoffatom: 5-Ring Heteroarylgruppen, welche neben Kohlenstoffatomen ein bis vier Stickstoffatome oder ein bis drei Stickstoffatome und ein Schwefel- oder Sauerstoffatom als Ringglieder enthalten können, z.B. 2-Furyl, 3-Furyl, 2-Thienyl, 3-Thienyl, 2-Pyrrolyl, 3-Pyrrolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, und 1,3,4-Triazol-2-yl;
   - 6-gliedriges Heteroaryl, enthaltend ein bis drei bzw. ein bis vier Stickstoffatome: 6-Ring Heteroarylgruppen, welche neben Kohlenstoffatomen ein bis drei bzw. ein bis vier Stickstoffatome als Ringglieder enthalten können, z.B. 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl und 2-Pyrazinyl;
Alkylen: divalente unverzweigte Ketten aus 3 bis 5 CH₂-Gruppen, z.B. CH₂, CH₂CH₂, CH₂CH₂CH₂, CH₂CH₂CH₂CH₂ und CH₂CH₂CH₂CH₂CH₂;
Oxyalkylen: divalente unverzweigte Ketten aus 2 bis 4 CH₂-Gruppen, wobei eine Valenz über ein Sauerstoffatom an das Gerüst gebunden ist, z.B. OCH₂CH₂, OCH₂CH₂CH₂ und OCH₂CH₂CH₂CH₂;
Oxyalkylenoxy: divalente unverzweigte Ketten aus 1 bis 3 CH₂-Gruppen, wobei beide Valenzen über ein Sauerstoffatom an das Gerüst gebunden ist, z.B. OCH₂O, OCH₂CH₂O und OCH₂CH₂CH₂O;

In dem Umfang der vorliegenden Erfindung sind die (R)- und (S)-Isomere und die Razemate von Verbindungen der Formel I eingeschlossen, die chirale Zentren aufweisen.

Im Hinblick auf ihre bestimmungsgemäße Verwendung der Triazolopyrimidine der Formel I sind die folgenden Bedeutungen der Substituenten, und zwar jeweils für sich allein oder in Kombination, besonders bevorzugt:
Verbindungen der Formel I werden bevorzugt, in denen R¹ nicht Wasserstoff bedeutet.
Verbindungen I werden besonders bevorzugt, in denen R¹ für C₁-C₆-Alkyl, C₂-C₆-Alkenyl oder C₁-C₈-Halogenalkyl steht.
Verbindungen I sind bevorzugt, in denen R¹ für eine Gruppe A steht: 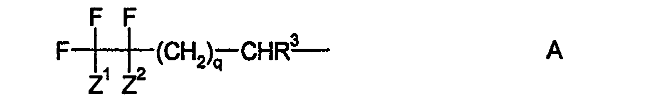
worin
- Z¹: Wasserstoff, Fluor oder C₁-C₆-Fluoroalkyl,
- Z²: Wasserstoff oder Fluor, oder
Z¹ und Z² bilden gemeinsam eine Doppelbindung;
- q: 0 oder 1 ist; und
- R³: Wasserstoff oder Methyl bedeuten.

Außerdem werden Verbindungen I bevorzugt, in denen R¹ für C₃-C₆-Cycloalkyl steht, welches durch C₁-C₄-Alkyl substituiert sein kann.

Insbesondere werden Verbindungen I bevorzugt, in denen R² Wasserstoff bedeutet.

Gleichermaßen bevorzugt sind Verbindungen I, in denen R² für Methyl oder Ethyl steht.

Sofern R¹ und/oder R² Halogenalkyl oder Halogenalkenylgruppen mit Chiralitätszentrum beinhalten, sind für diese Gruppen die (S)- Isomere bevorzugt. Im Fall halogenfreier Alkyl oder Alkenylgruppen mit Chiralitätszentrum in R¹ oder R² sind die (R)-konfigurierten Isomere bevorzugt.

Weiterhin werden Verbindungen I bevorzugt, in denen R¹ und R² zusammen mit dem Stickstoffatom, an das sie gebunden sind, einen Piperidinyl-, Morpholinyl- oder Thiomorpholinylring bilden, insbesondere einen Piperidinylring, der ggf. durch eine bis drei Gruppen Halogen, C₁-C₄-Alkyl oder C₁-C₄-Halogenalkyl substituiert ist. Besonders bevorzugt sind die Verbindungen, in denen R¹ und R² zusammen mit dem Stickstoffatom, an das sie gebunden sind, einen 2-Methyl-, 3-Methyl- oder einen 3,5-Dimethylpiperidinring bilden.

Daneben sind Verbindungen 1 bevorzugt, in denen R¹ und R² zusammen mit dem Stickstoffatom, an das sie gebunden sind, einen der vorgenannten fünfgliederigen gesättigten oder ungesättigten Ringe oder einen Morpholinyl- oder Thiomorpholinylring bilden, wobei die Ringe durch eine bis drei Gruppen Halogen, C₁-C₄-Alkyl oder C₁-C₄-Halogenalkyl substituiert sein können.

Ein besonders bevorzugter Gegenstand der Erfindung sind Verbindungen I, in denen R¹ und R² zusammen mit dem Stickstoffatom, an das sie gebunden sind, einen Pyrazol, oder Pyrrolidinring bilden, welche durch eine oder zwei Gruppen Halogen, C₁-C₄-Alkyl oder C₁-C₄-Halogenalkyl substituiert sein können, insbesondere 3,5-Dimethylpyrazol, 3,5-Di-(trifluormethyl)pyrazol, 2-Methylpyrrolidin oder 3-Methylpyrrolidin.

Daneben sind auch Verbindungen der Formel I bevorzugt, in denen R¹ CH(CH₃)-CH₂CH₃, CH(CH₃)-CH(CH₃)₂ , CH(CH₃)-C(CH₃)₃, CH(CH₃)-CF₃, CH₂C(CH₃)=CH₂ ,CH₂CH=CH₂, Cyclopentyl oder Cyclohexyl; R² Wasserstoff oder Methyl; oder R¹ und R² gemeinsam, -(CH₂)₂CH(CH₃)(CH₂)₂-, -CH(CH₃)(CH₂)₄-, -(CH₂)₂CH(CF₃)(CH₂)₂- oder -(CH₂)₂O(CH₂)₂- bedeuten.

Verbindungen I werden bevorzugt, in denen X C₁-C₄-Alkyl, Cyano oder C₁-C₄-Alkoxy, wie Methyl, Cyano, Methoxy oder Ethoxy, besonders Methyl bedeutet.

Eine bevorzugte Ausgestaltung der Erfindung betrifft Verbindungen der Formel I, in der L für Methyl steht.

Eine weitere bevorzugte Ausgestaltung der Erfindung betrifft Verbindungen der Formel I, in der L für Chlor steht.

Eine weitere bevorzugte Ausführungsform der Erfindung betrifft Verbindungen der Formel I, die der Formel I.1 entsprechen: 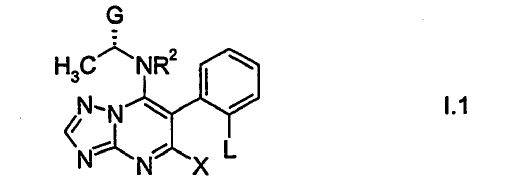 in der
- G: C₂-C₆-Alkyl, insbesondere Ethyl, n- und i-Propyl, n-, sek-, tert- Butyl, und C₁-C₄-Alkoxymethyl, insbesondere Ethoxymethyl, oder C₃-C₆-Cycloalkyl, insbesondere Cyclopentyl oder Cyclohexyl;
- R²: Wasserstoff oder Methyl; und
- X: Methyl, Cyano, Methoxy oder Ethoxy bedeuten.

Eine weitere bevorzugte Ausführungsform der Erfindung betrifft Verbindungen der Formel I, in denen R¹ und R² zusammen mit dem Stickstoffatom, an das sie gebunden sind, ein fünf- oder sechsgliedriges Heterocyclyl oder Heteroaryl bilden, welches über N gebunden ist und ein weiteres Heteroatom aus der Gruppe O, N und S als Ringglied enthalten und/oder einen oder mehrere Substituenten aus der Gruppe Halogen, C₁-C₆-Alkyl,
C₁-C₆-Halogenalkyl, C₂-C₆-Alkenyl, C₇C₆-Halogenalkenyl, C₁-C₆-Alkoxy, C₁-C₆-Halogenalkoxy, C₃-C₆-Alkenyloxy, C₃-C₆-Halogenalkenyloxy, C₁-C₆-Alkylen und Oxy-C₁-C₃-alkylenoxy tragen kann, wobei X nicht Methyl bedeutet, wenn D n-Pentylen oder 3-Methyl-n-pentylen und L Fluor bedeutet, oder D 3-Methyl-n-pentylen und L Chlor bedeutet. Diese Verbindungen entsprechen insbesondere Formel 1.2, 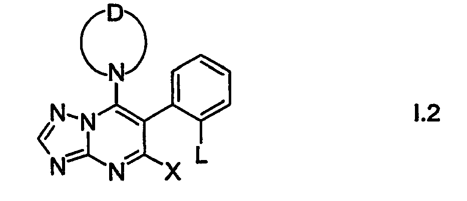 in der
- D: zusammen mit dem Stickstoffatom ein fünf- oder sechsgliedriges Heterocyclyl oder Heteroaryl bildet, welches über N gebunden ist und ein weiteres Heteroatom aus der Gruppe O, N und S als Ringglied enthalten und/oder einen oder mehrere Substituenten aus der Gruppe Halogen, C₁-C₄-Alkyl, C₁-C₄-Alkoxy und C₁-C₂-Halogenalkyl tragen kann; und
- X: Methyl, Cyano, Methoxy oder Ethoxy bedeuten,
wobei X nicht Methyl bedeutet, wenn D n-Pentylen oder 3-Methyl-n-pentylen und L Fluor bedeutet, oder D 3-Methyl-n-pentylen und L Chlor bedeutet.

Eine weitere bevorzugte Ausführungsform der Erfindung betrifft Verbindungen der Formel I, die der Formel 1.3 entsprechen: 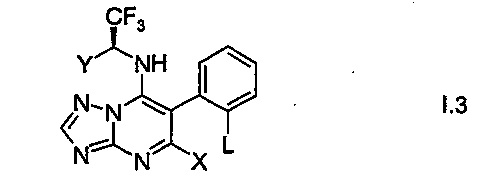 in der Y für Wasserstoff oder C₁-C₄-Alkyl, insbesondere für Methyl und Ethyl, und X für Methyl, Cyano, Methoxy oder Ethoxy steht.

Insbesondere sind im Hinblick auf ihre Verwendung die in den folgenden Tabellen zusammengestellten Verbindungen I bevorzugt. Die in den Tabellen für einen Substituenten genannten Gruppen stellen außerdem für sich betrachtet, unabhängig von der Kombination, in der sie genannt sind, eine besonders bevorzugte Ausgestaltung des betreffenden Substituenten dar.
Tabelle 1
   Verbindungen der Formel 1, in denen X Cyano, L Chlor und die Kombination von R¹ und R² für eine Verbindung jeweils einer Zeile der Tabelle A entspricht
Tabelle 2
   Verbindungen der Formel I, in denen X Methyl, L Chlor und die Kombination von R¹ und R² für eine Verbindung jeweils einer der Zeilen A-1 bis A-125 der Tabelle A entspricht
Tabelle 3
   Verbindungen der Formel I, in denen X Methoxy, L Chlor und die Kombination von R¹ und R² für eine Verbindung jeweils einer Zeile der Tabelle A entspricht
Tabelle 4
   Verbindungen der Formel I, in denen X Cyano, L Fluor und die Kombination von R¹ und R² für eine Verbindung jeweils einer Zeile der Tabelle A entspricht
Tabelle 5
   Verbindungen der Formel I, in denen X Methyl, L Fluor und die Kombination von R¹ und R² für eine Verbindung jeweils einer der Zeilen A-1 bis A-124 der Tabelle A entspricht
Tabelle 6
   Verbindungen der Formel 1, in denen X Methoxy, L Fluor und die Kombination von R¹ und R² für eine Verbindung jeweils einer Zeile der Tabelle A entspricht
Tabelle 7
   Verbindungen der Formel I, in denen X Cyano, L Methyl und die Kombination von R¹ und R² für eine Verbindung jeweils einer Zeile der Tabelle A entspricht
Tabelle 8
   Verbindungen der Formel I, in denen X Methyl, L Methyl und die Kombination von R¹ und R² für eine Verbindung jeweils einer Zeile der Tabelle A entspricht
Tabelle 9
   Verbindungen der Formel I, in denen X Methoxy, L Methyl und die Kombination von R¹ und R² für eine Verbindung jeweils einer Zeile der Tabelle A entspricht

**Tabelle A**

| **Nr.** | **R¹** | **R²** |
|---|---|---|
| A-1 | H | H |
| A-2 | CH₃ | H |
| A-3 | CH₃ | CH₃ |
| A-4 | CH₂CH₃ | H |
| A-5 | CH₂CH₃ | CH₃ |
| A-6 | CH₂CH₃ | CH₂CH₃ |
| A-7 | CH₂CF₃ | H |
| A-8 | CH₂CF₃ | CH₃ |
| A-9 | CH₂CF₃ | CH₂CH₃ |
| A-10 | CH₂CCl₃ | H |
| A-11 | CH₂CCl₃ | CH₃ |
| A-12 | CH₂CCl₃ | CH₂CH₃ |
| A-13 | CH₂CH₂CH₃ | H |
| A-14 | CH₂CH₂CH₃ | CH₃ |
| A-15 | CH₂CH₂CH₃ | CH₂CH₃ |
| A-16 | CH₂CH₂CH₃ | CH₂CH₂CH₃ |
| A-17 | CH(CH₃)₂ | H |
| A-18 | CH(CH₃)₂ | CH₃ |
| A-19 | CH(CH₃)₂ | CH₂CH₃ |
| A-20 | CH₂CH₂CH₂CH₃ | H |
| A-21 | CH₂CH₂CH₂CH₃ | CH₃ |
| A-22 | CH₂CH₂CH₂CH₃ | CH₂CH₃ |
| A-23 | CH₂CH₂CH₂CH₃ | CH₂CH₂CH₃ |
| A-24 | CH₂CH₂CH₂CH₃ | CH₂CH₂CH₂CH₃ |
| A-25 | (±) CH(CH₃)-CH₂CH₃ | H |
| A-26 | (±) CH(CH₃)-CH₂CH₃ | CH₃ |
| A-27 | (±) CH(CH₃)-CH₂CH₃ | CH₂CH₃ |
| A-28 | (S) CH(CH₃)-CH₂CH₃ | H |
| A-29 | (S) CH(CH₃)-CH₂CH₃ | CH₃ |
| A-30 | (S) CH(CH₃)-CH₂CH₃ | CH₂CH₃ |
| A-31 | (R) CH(CH₃)-CH₂CH₃ | H |
| A-32 | (R) CH(CH₃)-CH₂CH₃ | CH₃ |
| A-33 | (R) CH(CH₃)-CH₂CH₃ | CH₂CH₃ |
| A-34 | (±) CH(CH₃)-CH(CH₃)₂ | H |
| A-35 | (±) CH(CH₃)-CH(CH₃)₂ | CH₃ |
| A-36 | (±) CH(CH₃)-CH(CH₃)₂ | CH₂CH₃ |
| A-37 | (S) CH(CH₃)-CH(CH₃)₂ | H |
| A-38 | (S) CH(CH₃)-CH(CH₃)₂ | CH₃ |
| A-39 | (S) CH(CH₃)-CH(CH₃)₂ | CH₂CH₃ |
| A-40 | (R) CH(CH₃)-CH(CH₃)₂ | H |
| A-41 | (R) CH(CH₃)-CH(CH₃)₂ | CH₃ |
| A-42 | (R) CH(CH₃)-CH(CH₃)₂ | CH₂CH₃ |
| A-43 | (±) CH(CH₃)-C(CH₃)₃ | H |
| A-44 | (±) CH(CH₃)-C(CH₃)₃ | CH₃ |
| A-45 | (±) CH(CH₃)-C(CH₃)₃ | CH₂CH₃ |
| A-46 | (S) CH(CH₃)-C(CH₃)₃ | H |
| A-47 | (S) CH(CH₃)-C(CH₃)₃ | CH₃ |
| A-48 | (S) CH(CH₃)-C(CH₃)₃ | CH₂CH₃ |
| A-49 | (R) CH(CH₃)-C(CH₃)₃ | H |
| A-50 | (R) CH(CH₃)-C(CH₃)₃ | CH₃ |
| A-51 | (R) CH(CH₃)-C(CH₃)₃ | CH₂CH₃ |
| A-52 | (±) CH(CH₃)-CF₃ | H |
| A-53 | (±) CH(CH₃)-CF₃ | CH₃ |
| A-54 | (±) CH(CH₃)-CF₃ | CH₂CH₃ |
| A-55 | (S) CH(CH₃)-CF₃ | H |
| A-56 | (S) CH(CH₃)-CF₃ | CH₃ |
| A-57 | (S) CH(CH₃)-CF₃ | CH₂CH₃ |
| A-58 | (R) CH(CH₃)-CF₃ | H |
| A-59 | (R) CH(CH₃)-CF₃ | CH₃ |
| A-60 | (R) CH(CH₃)-CF₃ | CH₂CH₃ |
| A-61 | (±) CH(CH₃)-CCl₃ | H |
| A-62 | (±) CH(CH₃)-CCl₃ | CH₃ |
| A-63 | (±) CH(CH₃)-CCl₃ | CH₂CH₃ |
| A-64 | (S) CH(CH₃)-CCl₃ | H |
| A-65 | (S) CH(CH₃)-CCl₃ | CH₃ |
| A-66 | (S) CH(CH₃)-CCl₃ | CH₂CH₃ |
| A-67 | (R) CH(CH₃)-CCl₃ | H |
| A-68 | (R) CH(CH₃)-CCl₃ | CH₃ |
| A-69 | (R) CH(CH₃)-CCl₃ | CH₂CH₃ |
| A-70 | CH₂CF₂CF₃ | H |
| A-71 | CH₂CF₂CF₃ | CH₃ |
| A-72 | CH₂CF₂CF₃ | CH₂CH₃ |
| A-73 | CH₂(CF₂)₂CF₃ | H |
| A-74 | CH₂(CF₂)₂CF₃ | CH₃ |
| A-75 | CH₂(CF₂)₂CF₃ | CH₂CH₃ |
| A-76 | CH₂C(CH₃)=CH₂ | H |
| A-77 | CH₂C(CH₃)=CH₂ | CH₃ |
| A-78 | CH₂C(CH₃)=CH₂ | CH₂CH₃ |
| A-79 | CH₂CH=CH₂ | H |
| A-80 | CH₂CH=CH₂ | CH₃ |
| A-81 | CH₂CH=CH₂ | CH₂CH₃ |
| A-82 | CH(CH₃)CH=CH₂ | H |
| A-83 | CH(CH₃)CH=CH₂ | CH₃ |
| A-84 | CH(CH₃)CH=CH₂ | CH₂CH₃ |
| A-85 | CH(CH₃)C(CH₃)=CH₂ | H |
| A-86 | CH(CH₃)C(CH₃)=CH₂ | CH₃ |
| A-87 | CH(CH₃)C(CH₃)=CH₂ | CH₂CH₃ |
| A-88 | CH₂-C≡CH | H |
| A-89 | CH₂-C≡CH | CH₃ |
| A-90 | CH₂-C≡CH | CH₂CH₃ |
| A-91 | Cyclopentyl | H |
| A-92 | Cyclopentyl | CH₃ |
| A-93 | Cyclopentyl | CH₂CH₃ |
| A-94 | Cyclohexyl | H |
| A-95 | Cyclohexyl | CH₃ |
| A-96 | Cyclohexyl | CH₂CH₃ |
| A-97 | CH₂-C₆H₅ | H |
| A-98 | CH₂-C₆H₅ | CH₃ |
| A-99 | CH₂-C₆H₅ | CH₂CH₃ |
| A-100 | -(CH₂)₂CH=CHCH₂- | |
| A-101 | -(CH₂)₂C(CH₃)=CHCH₂- | |
| A-102 | -CH(CH₃)CH₂-CH=CHCH₂- | |
| A-103 | -(CH₂)₃CHFCH₂- | |
| A-104 | -(CH₂)₂CHF(CH₂)₂- | |
| A-105 | -CH₂CHF(CH₂)₃- | |
| A-106 | -(CH₂)₂CH(CF₃)(CH₂)₂- | |
| A-107 | -(CH₂)₂O(CH₂)₂- | |
| A-108 | -(CH₂)₂S(CH₂)₂- | |
| A-109 | -(CH₂)₄- | |
| A-110 | -CH₂CH=CHCH₂- | |
| A-111 | -CH(CH₃)(CH₂)₃- | |
| A-112 | -CH₂CH(CH₃)(CH₂)₂- | |
| A-113 | -CH(CH₃)-(CH₂)₂-CH(CH₃)- | |
| A-114 | -CH(CH₃)-(CH₂)₄- | |
| A-115 | -CH₂-CH(CH₃)-(CH₂)₃- | |
| A-116 | -(CH₂)-CH(CH₃)-CH₂-CH(CH₃)-CH₂- | |
| A-117 | -CH(CH₂CH₃)-(CH₂)₄- | |
| A-118 | -(CH₂)₆- | |
| A-119 | -CH(CH₃)-(CH₂)₅- | |
| A-120 | -(CH₂)₂-N(CH₃)-(CH₂)₂- | |
| A-121 | -N=CH-CH=CH- | |
| A-122 | -N=C(CH₃)-CH=C(CH₃)- | |
| A-123 | -N=C(CF₃)-CH=C(CF₃)- | |
| A-124 | -(CH₂)₂CH(CH₃)(CH₂)₂- | |
| A-125 | -(CH₂)₅- | |

Die Verbindungen I eignen sich als Fungizide. Sie zeichnen sich aus durch eine hervorragende Wirksamkeit gegen ein breites Spektrum von pflanzenpathogenen Pilzen, insbesondere aus der Klasse der *Ascomyceten, Deuteromyceten, Oomyceten* und Ba*sidiomyceten.* Sie sind zum Teil systemisch wirksam und können im Pflanzenschutz als Blatt-, Beiz- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Bananen, Baumwolle, Soja, Kaffee, Zuckerrohr, Wein, Obst- und Zierpflanzen und Gemüsepflanzen wie Gurken, Bohnen, Tomaten, Kartoffeln und Kürbisgewächsen, sowie an den Samen dieser Pflanzen.

Speziell eignen sie sich zur Bekämpfung folgender Pflanzenkrankheiten:
- *Alternaria-*Arten an Gemüse und Obst,
- *Bipolaris-* und *Drechslera-*Arten an Getreide, Reis und Rasen,
- *Blumeria graminis* (echter Mehltau) an Getreide,
- *Botrytis cinerea* (Grauschimmel) an Erdbeeren, Gemüse, Zierpflanzen und Reben,
- *Erysiphe cichoracearum* und *Sphaerotheca fuliginea* an Kürbisgewächsen,
- *Fusarium-* und *Verticillium-*Arten an verschiedenen Pflanzen,
- *Mycosphaerella-*Arten an Getreide, Bananen und Erdnüssen,
- *Phakopsora pachyrhizi* und *P. meibomiae* an Soja,
- *Phytophthora infestans* an Kartoffeln und Tomaten,
- *Plasmopara viticola* an Reben,
- *Podosphaera leucotricha* an Äpfeln,
- *Pseudocercosporella herpotrichoides* an Weizen und Gerste,
- *Pseudoperonospora-Arten* an Hopfen und Gurken,
- *Puccinia-Arten* an Getreide,
- *Pyricularia oryzae* an Reis,
- *Rhizoctonia-Arten* an Baumwolle, Reis und Rasen,
- *Septoria tritici* und *Stagonospora nodorum* an Weizen,
- *Uncinula necator* an Reben,
- Ustilago-Arten an Getreide und Zuckerrohr, sowie
- *Venturia-Arten* (Schorf) an Äpfeln und Birnen.

Die Verbindungen I eignen sich außerdem zur Bekämpfung von Schadpilzen wie Pae*cilomyces variotii* im Materialschutz (z.B. Holz, Papier, Dispersionen für den Anstrich, Fasern bzw. Gewebe) und im Vorratsschutz.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung kann sowohl vor als auch nach der Infektion der Materialien, Pflanzen oder Samen durch die Pilze erfolgen.

Die fungiziden Mittel enthalten im allgemeinen zwischen 0,1 und 95, vorzugsweise zwischen 0,5 und 90 Gew.-% Wirkstoff.

Die Aufwandmengen liegen bei der Anwendung im Pflanzenschutz je nach Art des gewünschten Effektes zwischen 0,01 und 2,0 kg Wirkstoff pro ha.

Bei der Saatgutbehandlung werden im allgemeinen Wirkstoffmengen von 1 bis 1000 g/100 kg Saatgut, vorzugsweise 1 bis 200 g/100 kg, insbesondere 5 bis 100 g/100 kg verwendet.

Bei der Anwendung im Material- bzw. Vorratsschutz richtet sich die Aufwandmenge an Wirkstoff nach der Art des Einsatzgebietes und des gewünschten Effekts. Übliche Aufwandmengen sind im Materialschutz beispielsweise 0,001 g bis 2 kg, vorzugsweise 0,005 g bis 1 kg Wirkstoff pro Kubikmeter behandelten Materials.

Die Verbindungen I können in die üblichen Formulierungen überführt werden, z.B. Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindung gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln. Als Lösungsmittel / Hilfsstoffe kommen dafür im wesentlichen in Betracht
- Wasser, aromatische Lösungsmittel (z.B. Solvesso Produkte, Xylol), Paraffine (z.B. Erdölfraktionen), Alkohole (z.B. Methanol, Butanol, Pentanol, Benzylalkohol), Ketone (z.B. Cyclohexanon, gamma-Butryolacton), Pyrrolidone (NMP, NOP), Acetate (Glykoldiacetat), Glykole, Dimethylfettsäureamide, Fettsäuren und Fettsäureester. Grundsätzlich können auch Lösungsmittelgemische verwendet werden,
- Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate, Fettsäuren und sulfatierte Fettalkoholglykolether zum Einsatz, ferner Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Tristerylphenylpolyglykolether, Alkylarylpolyetheralkohole, Alkohol- und Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Isophoron, stark polare Lösungsmittel, z.B. Dimethylsulfoxid, N-Methylpyrrolidon oder Wasser in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,01 und 95 Gew.-%, vorzugsweise zwischen 0,1 und 90 Gew.% des Wirkstoffs. Die Wirkstoffe werden dabei in einer Reinheit von 90% bis 100%, vorzugsweise 95% bis 100% (nach NMR-Spektrum) eingesetzt.

### Beispiele für Formulierungen sind: 1. Produkte zur Verdünnung in Wasser

### A Wasserlösliche Konzentrate (SL)

10 Gew.-Teile einer erfindungsgemäßen Verbindung werden in Wasser oder einem wasserlöslichen Lösungsmittel gelöst. Alternativ werden Netzmittel oder andere Hilfsmittel zugefügt. Bei der Verdünnung in Wasser löst sich der Wirkstoff.

### B Dispergierbare Konzentrate (DC)

20 Gew.-Teile einer erfindungsgemäßen Verbindung werden in Cyclohexanon unter Zusatz eines Dispergiermittels z.B. Polyvinylpyrrolidon gelöst. Bei Verdünnung in Wasser ergibt sich eine Dispersion.

### C Emulgierbare Konzentrate (EC)

15 Gew.-Teile einer erfindungsgemäßen Verbindung werden in Xylol unter Zusatz von Ca-Dodecylbenzolsulfonat und Ricinusölethoxylat (jeweils 5 %) gelöst. Bei der Verdünnung in Wasser ergibt sich eine Emulsion.

### D Emulsionen (EW, EO)

40 Gew.-Teile einer erfindungsgemäßen Verbindung werden in Xylol unter Zusatz von Ca-Dodecylbenzolsulfonat und Ricinusölethoxylat (jeweils 5 %) gelöst. Diese Mischung wird mittels einer Emulgiermaschine (Ultraturax) in Wasser eingebracht und zu einer homogenen Emulsion gebracht. Bei der Verdünnung in Wasser ergibt sich eine Emulsion.

### E Suspensionen (SC, OD)

20 Gew.-Teile einer erfindungsgemäßen Verbindung werden unter Zusatz von Dispergier- und Netzmitteln und Wasser oder einem organischen Lösungsmittel in einer Rührwerkskugelmühle zu einer feinen Wirkstoffsuspension zerkleinert. Bei der Verdünnung in Wasser ergibt sich eine stabile Suspension des Wirkstoffs.

### F Wasserdispergierbare und wasserlösliche Granulate (WG, SG)

50 Gew.-Teile einer erfindungsgemäßen Verbindung werden unter Zusatz von Dispergier- und Netzmitteln fein gemahlen und mittels technischer Geräte (z.B. Extrusion, Sprühturm, Wirbelschicht) als wasserdispergierbare oder wasserlösliche Granulate hergestellt. Bei der Verdünnung in Wasser ergibt sich eine stabile Dispersion oder Lösung des Wirkstoffs.

### G Wasserdispergierbare und wasserlösliche Pulver (WP, SP)

75 Gew.-Teile einer erfindungsgemäßen Verbindung werden unter Zusatz von Dispergier- und Netzmitteln sowie Kieselsäuregel in einer Rotor-Strator Mühle vermahlen. Bei der Verdünnung in Wasser ergibt sich eine stabile Dispersion oder Lösung des Wirkstoffs.

### 2. Produkte für die Direktapplikation

### H Stäube (DP)

5 Gew.Teile einer erfindungsgemäßen Verbindung werden fein gemahlen und mit 95 % feinteiligem Kaolin innig vermischt. Man erhält dadurch ein Stäubemittel.

### I Granulate (GR, FG, GG, MG)

0.5 Gew-Teile einer erfindungsgemäßen Verbindung werden fein gemahlen und mit 95.5 % Trägerstoffe verbunden. Gängige Verfahren sind dabei die Extrusion, die Sprühtrocknung oder die Wirbelschicht. Man erhält dadurch ein Granulat für die Direktapplikation.

### J ULV- Lösungen (UL)

10 Gew.-Teile einer erfindungsgemäßen Verbindung werden in einem organischen Lösungsmittel z.B. Xylol gelöst. Dadurch erhält man ein Produkt für die Direktapplikation.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Wässrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Die Wirkstoffkonzentrationen in den anwendungsfertigen Zubereitungen können in größeren Bereichen variiert werden. Im allgemeinen liegen sie zwischen 0,0001 und 10%, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Zu den Wirkstoffen können Öle verschiedenen Typs, Netzmittel, Adjuvants, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln. Beim Vermischen der Verbindungen I bzw. der sie enthaltenden Mittel in der Anwendungsform als Fungizide mit anderen Fungiziden erhält man in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
- Acylalanine wie Benalaxyl, Metalaxyl, Ofurace, Oxadixyl,
- Aminderivate wie Aldimorph, Dodine, Dodemorph, Fenpropimorph, Fenpropidin, Guazatine, Iminoctadine, Spiroxamin, Tridemorph
- Anilinopyrimidine wie Pyrimethanil, Mepanipyrim oder Cyrodinyl,
- Antibiotika wie Cycloheximid, Griseofulvin, Kasugamycin, Natamycin, Polyoxin oder Streptomycin,
- Azole wie Bitertanol, Bromoconazol, Cyproconazol, Difenoconazole, Dinitroconazol, Enilconazol, Epoxiconazol, Fenbuconazol, Fluquiconazol, Flusilazol, Flutriafol, Hexaconazol, Imazalil, Metconazol, Mydobutanil, Penconazol, Propiconazol, Prochloraz, Prothioconazol, Tebuconazol, Triadimefon, Triadimenol, Triflumizol, Triticonazol,
- Dicarboximide wie Iprodion, Myclozolin, Procymidon, Vinclozolin,
- Dithiocarbamate wie Ferbam, Nabam, Maneb, Mancozeb, Metam, Metiram, Propineb, Polycarbamat, Thiram, Ziram, Zineb,
- Heterocylische Verbindungen wie Anilazin, Benomyl, Boscalid, Carbendazim, Carboxin, Oxycarboxin, Cyazofamid, Dazomet, Dithianon, Famoxadon, Fenamidon, Fenarimol, Fuberidazol, Flutolanil, Furametpyr, Isoprothiolan, Mandipropamid, Mepronil, Nuarimol, Penthiopyrad, Probenazol, Proquinazid, Pyrifenox, Pyroquilon, Quinoxyfen, Silthiofam, Thiabendazol, Thifluzamid, Thiophanat-methyl, Tiadinil, Tricyclazol, Triforine,
- Kupferfungizide wie Bordeaux Brühe, Kupferacetat, Kupferoxychlorid, basisches Kupfersulfat,
- Nitrophenylderivate, wie Binapacryl, Dinocap, Dinobuton, Nitrophthal-isopropyl
- Phenylpyrrole wie Fenpiclonil oder Fludioxonil,
- Schwefel
- Sonstige Fungizide wie Acibenzolar-S-methyl, Benthiavalicarb, Carpropamid, Chlorothalonil, Cyflufenamid, Cymoxanil, Diclomezin, Diclocymet, Diethofencarb, Edifenphos, Ethaboxam, Fenhexamid, Fentin-Acetat, Fenoxanil, Ferimzone, Fluazinam, Fosetyl, Fosetyl-Aluminium, Iprovalicarb, Hexachlorbenzol, Metrafenon, Pencycuron, Propamocarb, Phosphorige Säure, Phthalid, Toloclofos-methyl, Quintozene, Zoxamid,
- Strobilurine wie Azoxystrobin, Dimoxystrobin, Fluoxastrobin, Kresoxim-methyl, Metominostrobin, Orysastrobin, Picoxystrobin, Pyraclostrobin oder Trifloxystrobin,
- Sulfensäurederivate wie Captafol, Captan, Dichlofluanid, Folpet, Tolylfluanid,
- Zimtsäureamide und Analoge wie Dimethomorph, Flumetover oder Flumorph.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in der anschließenden Tabelle mit physikalischen Daten aufgeführt.

### Beispiel 1 - Herstellung von 5-Methoxy-6-(2-chlor-phenyl)-7-(2-methyl-piperidin-1-yl-1,2,4-triazolo[1,5a]pyrimidin

### Stufe 1: 5-Chlor-6-(2-chlor-phenyl)-7-(2-methyl-piperidin-1-yl)-1,2,4-triazolo[1,5a]py rimidin

Eine Lösung von 1,5 g (5 mmol) 5,7-Dichlor-6-(2-chloro-phenyl)-1,2,4-triazolo[1,5a]pyrimidin (vgl. WO 03/80615), 0,5 g (5,1 mmol) Triethylamin und 0,5 g (5,1 mmol) 2-Methyl-piperidin in 10 ml Methylenchlorid wurden etwa 15 Std. bei 20-25°C gerührt. Anschließend wurde nach Zugabe von 0,05 g (0,51 mmol) Triethylamin und 0,05 g (0,51 mmol) 2-Methyl-piperidin weitere 20 Std. bei 20-25°C gerührt. Dann wurde die Reaktionsmischung mit verd. Salzsäure und Wasser extrahiert. Die organische Phase wurde nach Trocknung vom Lösungsmittel befreit. Es blieben erhielt 1,5 g 5-Chlor-6-(2-chlorphenyl)-7-(2-methyl-piperidin-1-yl)-1,2,4-triazolo[1,5a]pyrimidin (Reinheit (HPLC): 64%) als gelbes Öl zurück, das ohne weitere Reinigung in der nächsten Reaktion eingesetzt wurde.
¹H-NMR (CDCl₃, δ in ppm): 2 Rotamere ca. 2:1: 8,4 (2s, 1 H); 7,55 (m, 1 H); 7,2 - 7,5 (m, 3H); 4,6; 4,15 (2m, 1 H); 3,55; 3,1 (2m, 1 H); 3,35; 2,7 (2m, 1 H); 1,25 bis 1,9 (m, 6H); 1,15 (2d, 3H).

### Stufe 2: 5-Methoxy-6-(2-chlor-phenyl)-7-(2-methyl-piperidin-1-yl)-1,2,4-triazolo[1,5a]pyrimidin [I-1]

1,5 g der Verbindung aus Bsp. 1 (Reinheit ca. 64 %, ca. 2,7 mmol) und 1,2 g 30 %ige Natrium-Methanolat-Lsg. (6,7 mmol Natriummethanolat) in 20 ml Methanol wurden etwa 15 Std. bei 20-25°C gerührt. Anschließend wurde die Reaktionsmischung mit Wasser verdünnt, die wässrige Phase mit Methylenchlorid extrahiert. Die vereinigten organischen Phasen wurden mit Wasser gewaschen, getrocknet und vom Lösungsmittel befreit. Der Rückstand wurde mittels präparativer MPLC über Kieselgel RP-18 gereinigt. Man erhielt 0,4 g der Titelverbindung als farblosen Festkörper vom Fp 186-187°C).
¹H-NMR (CDCl₃, δ in ppm): 2 Rotamere ca. 3:1: 8,2 (s, breit, 1 H); 7,5 (m, 1 H); 7,2-7,4 (m, 3H); 4,5; 3,9 (2m, 1H); 4,0 (s, 3H); 3,4; 2,9 (2m, 1H); 3,3; 2,75 (2m, 1H); 1,15-1,9 (m, 6H); 1,15; 1,05 (2d, 3H).

**Tabelle I**

| **Nr.** | **R¹** | **R²** | **L** | **X** | **phys. Daten (Fp. [°C], ¹H-NMR [δ: ppm {CDCl₃}])** |
|---|---|---|---|---|---|
| I-1 | -CH(CH₃)-(CH₂)₄- | | Cl | OCH₃ | 186 -187 |
| I-2 | -(CH₂)₂-CH(CH₃)-(CH₂)₂- | | Cl | CH₃ | 207 - 208 |
| I-3 | -CH(CH₃)-(CH₂)₄- | | F | CN | 201 - 202 |
| I-4 | -CH(CH₃)-(CH₂)₄- | | F | OCH₃ | 170-172 |
| I-5 | -CH(CH₃)-(CH₂)₄- | | F | CH₃ | 160 -164 |
| I-6 | -CH(CH₃)-(CH₂)₄- | | Cl | CH₃ | 184 -186 |
| I-7 | (S) CH(CH₃)-CF₃ | H | Cl | CH₃ | 8,35 (s, 1H); 7,65 (m, 1 H); 7,5 (m, 2H); 7,35 (m, 1 H); 2,3, 2,25 (2s, 3H); 1,3, 1,25 (2d, 3H) |
| I-8 | (S) CH(CH₃)-CF₃ | H | F | CH₃ | 149-151 |
| I-9 | -CH(CH₃)-(CH₂)₄- | | CH₃ | CN | 176-178 |
| I-10 | -CH(CH₃-(CH₂)₄- | | CH₃ | OCH₃ | 211-213 |
| I-11 | -CH(CH₃)-(CH₂)₄- | | CH₃ | CH₃ | 182-184 |
| I-12 | (S)CH(CH₃)-CF₃ | H | CH₃ | CH₃ | 128-130 |
| I-13 | -(CH₂)₂-CH(CH₃)-(CH₂)₂- | | Cl | OC₂H₅ | 8,3 (s, 1 H); 7,5 (m, 1 H); 7,35 (m, 2H); 7,2 (m, 1 H); 4,5 (m, 1 H); 4,4 (m, 1 H); 0,95 (d, 3H) |
| I-14 | -(CH₂)₂-CH(CH₃)-(CH₂)₂- | | Cl | OCH₃ | 209-211 |

### Beispiele für die Wirkung gegen Schadpilze

Die fungizide Wirkung der Verbindungen der Formel I ließ sich durch die folgenden Versuche zeigen:

Die Wirkstoffe wurden getrennt als eine Stammlösung aufbereitet mit 25 mg Wirkstoff, welcher mit einem Gemisch aus Aceton und/oder DMSO und dem Emulgator Uniperol® EL (Netzmittel mit Emulgier- und Dispergierwirkung auf der Basis ethoxylierter Alkylphenole) im Volumen-Verhältnis Lösungsmittel-Emulgator von 99 zu 1 ad 10 ml aufgefüllt wurde. Anschließend wurde ad 100 ml mit Wasser aufgefüllt Diese Stammlösung wurde mit dem beschriebenen Lösungsmittel-Emulgator-Wasser Gemisch zu der unten angegeben Wirkstoffkonzentration verdünnt. Alternativ dazu wurden die Wirkstoffe als handelsübliche Fertigformulierung verwendet und mit Wasser auf die angegebene Wirkstoffkonzentration verdünnt.

Anwendungsbeispiel 1 - Wirksamkeit gegen die Dürrfleckenkrankheit der Tomate verursacht durch *Alternaria solani*

Blätter von Topfpflanzen der Sorte "Goldene Königin" wurden mit einer wässriger Suspension in der unten angegebenen Wirkstoffkonzentration bis zur Tropfnässe besprüht. Am folgenden Tag wurden die Blätter mit einer wässrigen Sporenaufschwemmung von *Altemaria solani* in 2 % Biomalzlösung mit einer Dichte von 0,17 x 10⁶ Sporen/ml infiziert. Anschließend wurden die Pflanzen in einer wasserdampf-gesättigten Kammer bei Temperaturen zwischen 20 und 22°C aufgestellt. Nach 5 Tagen hatte sich die Krankheit auf den unbehandelten, jedoch infizierten Kontrollpflanzen so stark entwickelt, dass der Befall visuell in % ermittelt.

In diesem Test zeigten die mit 250 ppm der Verbindungen I-2, I-5 bis I-9, I-11, I-12, bzw. I-14 behandelten Pflanzen nicht mehr als 5 % Befall, während die unbehandelten Pflanzen zu 90 % befallen waren.

Anwendungsbeispiel 2 - Wirksamkeit gegen den Grauschimmel an Paprikablättern verursacht durch *Botrytis cinerea* bei protektiver Anwendung

Paprikasämlinge der Sorte "Neusiedler Ideal Elite" wurden, nachdem sich 2-3 Blätter gut entwickelt hatten, mit einer wässrigen Suspension in der unten angegebenen Wirkstoffkonzentration bis zur Tropfnässe besprüht. Am nächsten Tag wurden die behandelten Pflanzen mit einer Sporensuspension von *Botrytis cinerea,* die 1,7 x 10⁶ Sporen/ml in einer 2 %igen wässrigen Biomalzlösung enthielt, inokuliert Anschließend wurden die Versuchspflanzen in eine Klimakammer mit 22 bis 24°C, Dunkelheit und hoher Luftfeuchtigkeit gestellt. Nach 5 Tagen konnte das Ausmaß des Pilzbefalls auf den Blättern visuell in % ermittelt werden.

In diesem Test zeigten die mit 250 ppm der Verbindungen I-2, I-5 bis I-9, I-11, I-12, I-13, bzw. I-14 behandelten Pflanzen nicht mehr als 20 % Befall, während die unbehandelten Pflanzen zu 100 % befallen waren.

## Patentansprüche

1. Triazolopyrimidine der Formel I 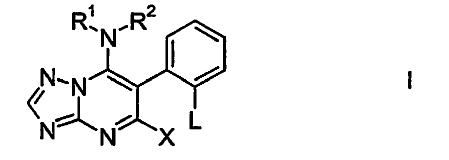 in der die Substituenten folgende Bedeutung haben:
R¹ C₁-C₈-Alkyl, C₁-C₈-Halogenalkyl, C₃-C₈-Cycloalkyl, C₃-C₈-Halogencycloalkyl, C₂-C₈-Alkenyl, C₂-C₈-Halogenalkenyl, C₃-C₆-Cycloalkenyl, C₃-C₆-Halogencycloalkenyl, C₂-C₈-Alkinyl, C₂-C₈-Halogenalkinyl oder Phenyl, Naphthyl, oder ein fünf- oder sechsgliedriger gesättigter, partiell ungesättigter oder aromatischer Heterocyclus, enthaltend ein bis vier Heteroatome aus der Gruppe O, N oder S,
R² Wasserstoff oder eine der bei R¹ genannten Gruppen,
R¹ und R² können auch zusammen mit dem Stickstoffatom, an das sie gebunden sind, ein fünf- oder sechsgliedriges Heterocyclyl oder Heteroaryl bilden, welches über N gebunden ist und ein bis drei weitere Heteroatome aus der Gruppe O, N und S als Ringglied enthalten und die einen oder mehrere Substituenten aus der Gruppe Halogen, C₁-C₆-Alkyl, C₁-C₆-Halogenalkyl, C₂-C₆-Alkenyl, C₂-C₆-Halogenalkenyl, C₁-C₆-Alkoxy, C₁-C₆-Halogenalkoxy, C₃-C₆-Alkenyloxy, C₃-C₆-Halogenalkenyloxy, (exo)-C₁-C₆-Alkylen und Oxy-C₁-C₃-alkylenoxy tragen kann;
R¹ und/oder R² können eine bis vier gleiche oder verschiedene Gruppen R^{a} tragen:
R^{a} Halogen, Cyano, Nitro, Hydroxy, C₁-C₆-Alkyl, C₁-C₆-Halogenalkyl, C₁-C₆-Alkylcarbonyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₈-Alkenyl, C₂-C₈-Halogenalkenyl, C₃-C₈-Cycloalkenyl, C₂-C₆-Alkenyloxy, C₃-C₆-Halogenalkenyloxy, C₂-C₆-Alkinyl, C₂-C₆-Halogenalkinyl, C₃-C₆-Alkinyloxy, C₃-C₆-Halogenalkinyloxy, C₃-C₆-Cycloalkoxy, C₃-C₆-Cycloalkenyloxy, Oxy-C₁-C₃-alkylenoxy, Phenyl, Naphthyl, fünf- bis zehngliedriger gesättigter, partiell ungesättigter oder aromatischer Heterocyclus, enthaltend ein bis vier Heteroatome aus der Gruppe O, N oder S,
wobei diese aliphatischen, alicyclischen oder aromatischen Gruppen ihrerseits partiell oder vollständig halogeniert sein oder eine bis drei Gruppen R^{b} tragen können:
R^{b} Halogen, Cyano, Nitro, Hydroxy, Mercapto, Amino, Carboxyl, Aminocarbonyl, Aminothiocarbonyl, Alkyl, Haloalkyl, Alkenyl, Alkenyloxy, Alkinyloxy, Alkoxy, Halogenalkoxy, Alkylthio, Alkylamino, Dialkylamino, Formyl, Alkylcarbonyl, Alkylsulfonyl, Alkylsulfoxyl, Alkoxycarbonyl, Alkylcarbonyloxy, Alkylaminocarbonyl, Dialkylaminocarbonyl, Alkylaminothiocarbonyl, Dialkylaminothiocarbonyl, wobei die Alkylgruppen in diesen Resten 1 bis 6 Kohlenstoffatome enthalten und die genannten Alkenyl- oder Alkinylgruppen in diesen Resten 2 bis 8 Kohlenstoffatome enthalten;
und/oder einen bis drei der folgenden Reste:
Cycloalkyl, Cycloalkoxy, Heterocyclyl, Heterocyclyloxy, wobei die cyclischen Systeme 3 bis 10 Ringglieder enthalten; Aryl, Aryloxy, Arylthio, Aryl-C₁-C₆-alkoxy, Aryl-C₁-C₆-alkyl, Hetaryl, Hetaryloxy, Hetarylthio, wobei die Arylreste vorzugsweise 6 bis 10 Ringglieder, die Hetarylreste 5 oder 6 Ringglieder enthalten, wobei die cyclischen Systeme partiell oder vollständig halogeniert oder durch Alkyl- oder Haloalkylgruppen substituiert sein können;
L Fluor, Chlor oder Methyl,
X Cyano, C₁-C₄-Alkyl, C₁-C₄-Alkoxy oder C₁-C₂-Halogenalkoxy;
wobei X nicht Methyl bedeutet, wenn R¹ und R² gemeinsam n-Pentylen oder 3-Methyl-n-pentylen und L Fluor bedeutet, oder R¹ und R² gemeinsam 3-Methyl-n-pentylen und L Chlor bedeutet

2. Verbindungen der Formel I gemäß Anspruch 1, mit der Maßgabe, dass R¹ und R² gemeinsam nicht Piperidin-1-yl oder 4-Alkyl-piperidin-1-yl bedeuten können.

3. Verbindungen der Formel I gemäß Anspruch 1, in der X Cyano, C₁-C₄-Alkoxy oder C₁-C₂-Halogenalkoxy bedeutet.

4. Verbindungen der Formel I gemäß Anspruch 1, in der X C₁-C₄-Alkyl bedeutet.

5. Verbindungen der Formel I gemäß einem der Ansprüche 1 bis 4, in der L Methyl bedeutet.

6. Verbindungen der Formel I gemäß einem der Ansprüche 1 bis 5, in der R¹ C₁-C₈-Alkyl, C₁-C₈-Halogenalkyl, C₃-C₈-Cycloalkyl, C₃-C₈-Halogencycloalkyl, C₂-C₈-Alkenyl, C₂-C₈-Halogenalkenyl, C₃-C₆-Cycloalkenyl, C₃-C₆-Halogencycloalkenyl, C₂-C₈-Alkinyl, C₂-C₈-Halogenalkinyl oder Phenyl, Naphthyl, oder ein fünf- oder sechsgliedriger gesättigter, partiell ungesättigter oder aromatischer Heterocyclus, enthaltend ein bis vier Heteroatome aus der Gruppe O, N oder S und R2 Wasserstoff oder C₁-C₄-Alkyl bedeutet, oder
zusammen mit dem Stickstoffatom, an das sie gebunden sind, ein fünfgliedriges Heterocyclyl oder Heteroaryl bilden, welches über N gebunden ist und ein bis drei weitere Heteroatome aus der Gruppe O, N und S als Ringglied enthalten, wobei R¹ und R² gemäß Anspruch 1 substitutiert sein können.

7. Verbindungen der Formel I gemäß einem der Ansprüche 1 bis 6, in der R² nicht Wasserstoff bedeutet.

8. Verbindungen der Formel I gemäß Anspruch 1, entsprechend Formel I.1: 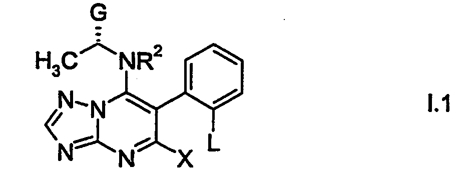 in der G C₂-C₆-Alkyl, C₁-C₄-Alkoxymethyl oder C₃-C₆-Cycloalkyl bedeuten.

9. Verbindungen der Formel I gemäß Anspruch 1, entsprechend Formel 1.2: 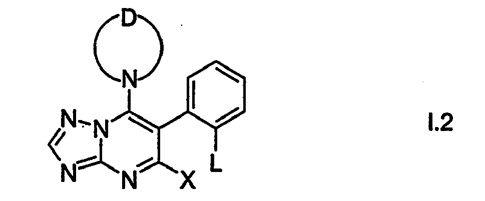 in der D zusammen mit dem Stickstoffatom ein fünf- oder sechsgliedriges Heterocyclyl oder Heteroaryl bildet, welches über N gebunden ist und ein weiteres Heteroatom aus der Gruppe O, N und S als Ringglied enthalten und/oder einen oder mehrere Substituenten aus der Gruppe Halogen, C₁-C₄-Alkyl, C₁-C₄-Alkoxy und C₁-C₂-Halogenalkyl tragen kann.

10. Verfahren zur Herstellung der Verbindungen der Formel I gemäß Anspruch 1, in der X für Cyano, C₁-C₄-Alkyl, C₁-C₄-Alkoxy oder C₁-C₂-Halogenalkoxy steht, durch Umsetzung von 5-Aminotriazol der Formel II 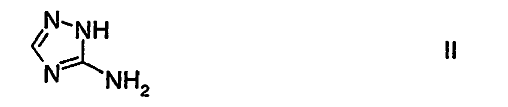 mit Phenymalonaten der Formel III, 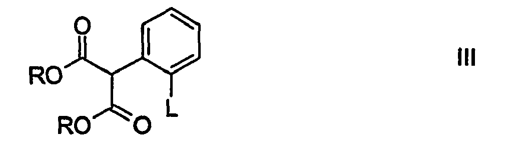 in der R für Alkyl steht, zu Dihydroxytriazolopyrimidinen der Formel IV, 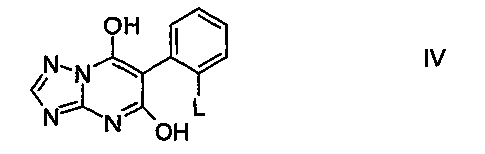 Halogenierung zu den Dihalogenverbindungen der Formel V, 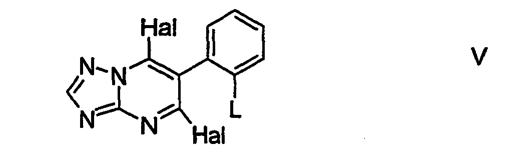 und Umsetzung von V mit Aminen der Formel VI 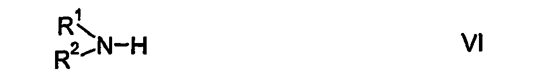 zu Verbindungen der Formel VII, 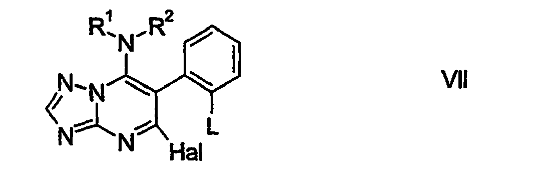 Umsetzung von Verbindungen VII mit Verbindungen der Formel VIII,
M-X' VIII
die, je nach der einzuführenden Gruppe X', ein anorganisches Cyanid, ein Alkoxylat oder ein Halogenalkoxylat darstellen und in der M für ein Ammonium-, Tetraalkylammonium-, Alkali- oder Erdaikatimetallkation steht und, gewünschtenfalls, zur Herstellung von Verbindungen der Formel 1 gemäß Anspruch 1, in der X für Alkyl steht, durch Umsetzung der Verbindungen VII mit Malonaten der Formel IX, 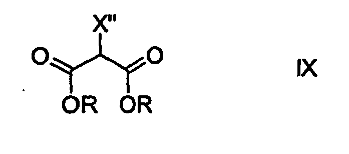 in der X" Wasserstoff oder C₁-C₃-Alkyl und R C₁-C₄-Alkyl bedeuten, zu Verbindungen der Formel X 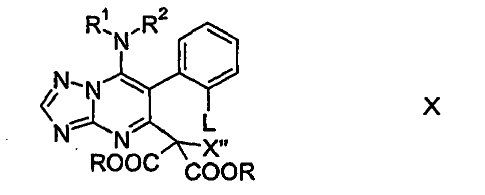 und Decarboxylierung zu Verbindungen 1, in denen X für Alkyl steht.

11. Verfahren zur Herstellung der Verbindungen der Formel I gemäß Anspruch 1, in der X für C₁-C₄-Alkyl oder C₁-C₄-Halogenalkyl steht, durch Umsetzung von 5-Aminotriazol der Formel II gemäß Anspruch 2 mit Ketoestem der Formel IIIa, 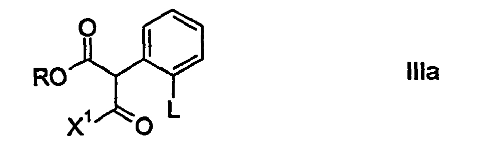 in der X¹ für C₁-C₄-Alkyl oder C₁-C₄Halogenalkyl und R für C₁-C₄-Alkyl steht, zu 5-Alkyl-7-hydroxy-6-phenyltriazolopyrimidinen der Formel IVa 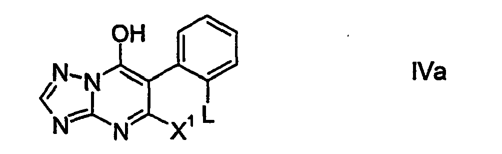 Halogenierung von IVa zu 7-Hatogenotriazolopyrimidinen der Formel Va 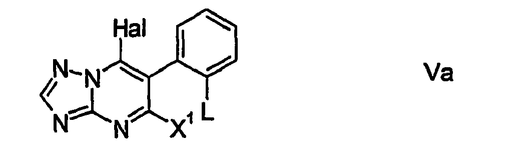 und Umsetzung von Va mit Aminen der Formel VI gemäß Anspruch 2 zu Verbindungen I, in denen X für C₁-C₄-Alkyl oder C₁-C₄-Halogenalkyl steht

12. Fungizides Mittel, enthaltend einen festen oder flüssigen Träger und eine Verbindung der Formel 1 gemäß Anspruch 1.

13. Saatgut, enthaltend 1 bis 1000 g einer Verbindung der Formel I gemäß Anspruch 1 pro 100 kg.

14. Verfahren zur Bekämpfung von pflanzenpathogenen Schadpilzen, **dadurch gekennzeichnet, dass** man die Pilze, oder die vor Pilzbefall zu schützenden Materialien, Pflanzen, den Boden oder Saatgüter mit einer wirksamen Menge einer Verbindung der Formel I gemäß Anspruch 1 behandelt.

## Claims

1. A triazolopyrimidine of the formula I 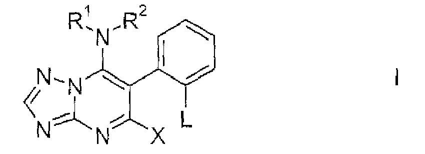 in which the substituents are as defined below:
R¹ is C₁-C₈-alkyl, C₁-C₈-haloalkyl, C₃-C₈-cycloalkyl, C₃-C₈-halocycloalkyl, C₂-C₈-alkenyl, C₂-C₈-haloalkenyl, C₃-C₆-cycloalkenyl, C₃-C₆-halocycloalkenyl, C₂-C₈-alkynyl, C₂-C₈-haloalkynyl or phenyl, naphthyl, or a five- or six-membered saturated, partially unsaturated or aromatic heterocycle comprising one to four heteroatoms from the group consisting of O, N and S,
R² is hydrogen or one of the groups mentioned under R¹,
R¹ and R² together with the nitrogen atom, to which they are attached, may also form a five- or six-membered heterocyclyl or heteroaryl which is attached via N and which may comprise one to three further heteroatoms from the group consisting of O, N and S as ring member and/or carry one or more substituents from the group consisting of halogen, C₁-C₆-alkyl, C₁-C₆-haloalkyl, C₂-C₆-alkenyl, C₂-C₆-haloalkenyl, C₁-C₆-alkoxy, C₁-C₆-haloalkoxy, C₃-C₆-alkenyloxy, C₃-C₆-haloalkenyloxy, (exo) -C₁-C₆-alkylene and oxy-C₁-C₃-alkyleneoxy;
R¹ and/or R² may carry one to four identical or different groups R^{a}:
R^{a} is halogen, cyano, nitro, hydroxyl, C₁-C₆-alkyl, C₁-C₆-haloalkyl, C₁-C₆-alkylcarbonyl, C₃-C₆-cycloalkyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₂-C₈-alkenyl, C₂-C₈-haloalkenyl, C₃-C₈-cycloalkenyl, C₂-C₆-alkenyloxy, C₃-C₆-haloalkenyloxy, C₂-C₆-alkynyl, C₂-C₆-haloalkynyl, C₃-C₆-alkynyloxy, C₃-C₆-haloalkynyloxy, C₃-C₆-cycloalkoxy, C₃-C₆-cycloalkenyloxy, oxy-C₁-C₃-alkyleneoxy, phenyl, naphthyl, a five- to ten-membered saturated, partially unsaturated or aromatic heterocycle comprising one to four heteroatoms from the group consisting of 0, N and S,
where these aliphatic, alicyclic or aromatic groups for their part may be partially or fully halogenated or may carry one to three groups R^{b}:
R^{b} is halogen, cyano, nitro, hydroxyl, mercapto, amino, carboxyl, aminocarbonyl, aminothiocarbonyl, alkyl, haloalkyl, alkenyl, alkenyloxy, alkynyloxy, alkoxy, haloalkoxy, alkylthio, alkylamino, dialkylamino, formyl, alkylcarbonyl, alkylsulfonyl, alkylsulfoxyl, alkoxycarbonyl, alkylcarbonyloxy, alkylaminocarbonyl, dialkylaminocarbonyl, alkylaminothiocarbonyl, dialkylaminothiocarbonyl, where the alkyl groups in these radicals comprise 1 to 6 carbon atoms and the alkenyl or alkynyl groups mentioned in these radicals comprise 2 to 8 carbon atoms;
and/or one to three of the following radicals:
cycloalkyl, cycloalkoxy, heterocyclyl, heterocyclyloxy, where the cyclic systems comprise 3 to 10 ring members; aryl, aryloxy, arylthio, aryl-C₁-C₆-alkoxy, aryl-C₁-C₆-alkyl, hetaryl, hetaryloxy, hetarylthio, where the aryl radicals preferably comprise 6 to 10 ring members and the hetaryl radicals comprise 5 or 6 ring members, where the cyclic systems may be partially or fully halogenated or substituted by alkyl or haloalkyl groups;
L is fluorine, chlorine or methyl,
x is cyano, C₁-C₄-alkyl, C₁-C₄-alkoxy or C₁-C₂-haloalkoxy;
where X is not methyl if R¹ and R² together are n-pentylene or 3-methyl-n-pentylene and L is fluorine, or R¹ and R² together are 3-methyl-n-pentylene and L is chlorine.

2. The compound of the formula I according to claim 1, with the proviso that R¹ and R² together may not be piperidin-1-yl or 4-alkylpiperidin-1-yl.

3. The compound of the formula I according to claim 1 in which X is cyano, C₁-C₄-alkoxy or C₁-C₂-haloalkoxy.

4. The compound of the formula I according to claim 1 in which X is C₁-C₄-alkyl.

5. The compound of the formula I according to any of claims 1 to 4 in which L is methyl.

6. The compound of the formula I according to any of claims 1 to 5 in which R¹ is C₁-C₈-alkyl, C₁-C₈-haloalkyl, C₃-C₈-cycloalkyl, C₃-C₈-halocycloalkyl, C₂-C₈-alkenyl, C₂-C₈-haloalkenyl, C₃-C₆-cycloalkenyl, C₃-C₆-halocycloalkenyl, C₂-C₈-alkynyl, C₂-C₈-halogenalkynyl oder phenyl, naphthyl, or a five- or six-membered saturated, partially unsaturated or aromatic heterocycle comprising one to four heteroatoms from the group consisting of O, N and S and R² is hydrogen or C₁-C₄-alkyl, or together with the nitrogen atom, to which they are attached, form a five- or six-membered heterocyclyl or heteroaryl which is attached via N and which may comprise one to three further heteroatoms from the groups consisting of O, N and S as ring member, where R¹ and R² may be substituted according to claim 1.

7. The compound of the formula I according to any of claims 1 to 6 in which R² is not hydrogen.

8. The compound of the formula I according to claim 1 corresponding to the formula I.1: 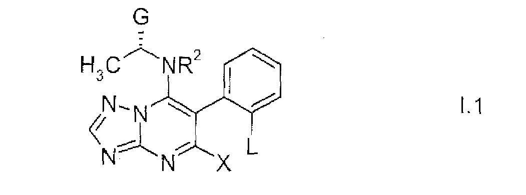 in which G is C₂-C₆-alkyl, C₁-C₄-alkoxymethyl or C₃-C₆-cycloalkyl.

9. The compound of the formula I according to claim 1 corresponding to the formula I.2: 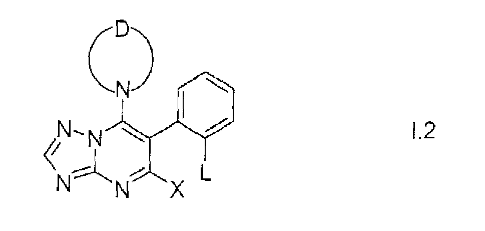 in which D together with the nitrogen atom form a five- or six-membered heterocyclyl or heteroaryl which is attached via N and may comprise a further heteroatom from the group consisting of 0, N and S as ring member and/or may carry one or more substituents from the group consisting of halogen, C₁-C₄-alkyl, C₁-C₄-alkoxy and C₁-C₂-haloalkyl.

10. A process for preparing compounds of the formula I according to claim 1 in which X is cyano, C₁-C₄-alkyl, C₁-C₄-alkoxy or C₁-C₂-haloalkoxy by reaction of 5-aminotriazole of the formula II 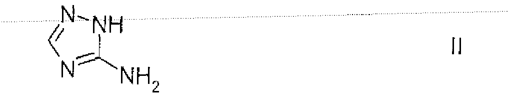 with phenylmalonates of the formula III 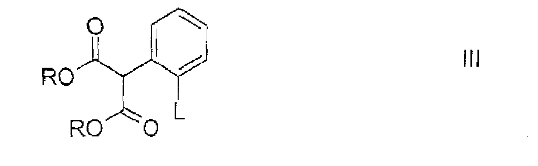 in which R is alkyl to give dihydroxytriazolopyrimidines of the formula IV, 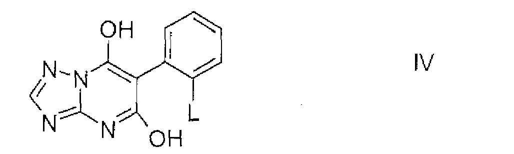 halogenation to give the dihalo compounds of the formula V 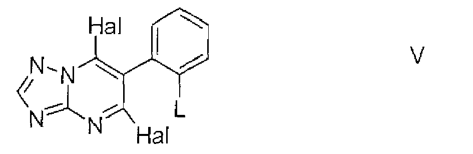 and reaction of V with amines of the formula VI 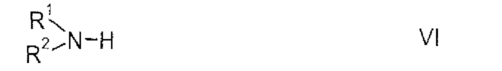 to give compounds of the formula VII, 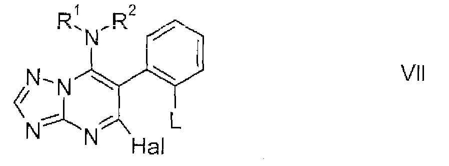 reaction of compounds VII with compounds of the formula VIII
M-X' VIII
which, depending on the group X' to be introduced, are inorganic cyanides, alkoxides or haloalkoxides and in which M is an ammonium, tetraalkylammonium, alkali metal or alkaline earth metal cation, and, if desired, for preparing compounds of the formula I as claimed in claim 1 in which X is alkyl by reaction of the compounds VII with malonates of the formula 1X 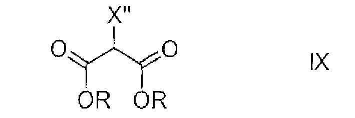 in which X" is hydrogen or C₁-C₃-alkyl and R is C₁-C₄-alkyl to give compounds of the formula X 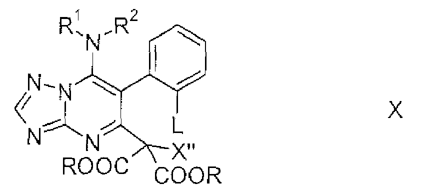 and decarboxylation to give compounds I in which X is alkyl.

11. A process for preparing compounds of the formula I according to claim 1 in which X is C₁-C₄-alkyl or C₁-C₄-haloalkyl by reaction of 5-aminotriazole of the formula II according to claim 10 with keto esters of the formula IIIa 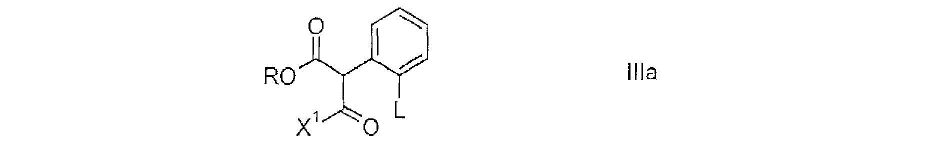 in which X¹ is C₁-C₄-alkyl or C₁-C₄-haloalkyl and R is C₁-C₄-alkyl to give 5-alkyl-7-hydroxy-6-phenyltriazolopyrimidines of the formula IVa, 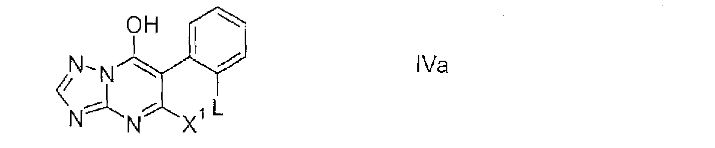 halogenation of IVa to give 7-halotriazolo-pyrimidines of the formula Va 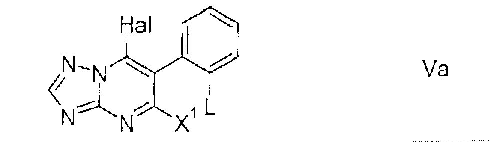 and reaction of Va with amines of the formula VI according to claim 10 to give compounds I in which X is C₁-C₄-alkyl or C₁-C₄-haloalkyl.

12. A fungicidal composition comprising a solid or liquid carrier and a compound of the formula I according to claim 1.

13. Seed comprising from 1 to 1000 g of a compound of the formula I according to claim 1 per 100 kg.

14. A method for controlling phytopathogenic harmful fungi, which method comprises treating the fungi or the materials, plants, the soil or seed to be protected against fungal attack with an effective amount of a compound of the formula I according to claim 1.

## Revendications

1. Triazolopyrimidines de formule I 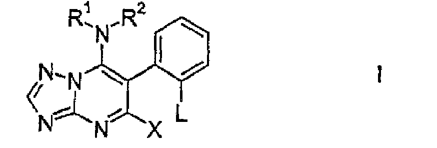 dans laquelle les substituants ont la signification suivante :
R¹ signifie un alkyle en C₁-C₈, un halogénoalkyle en C₁-C₈, un cycloalkyle en C₃-C₈, un halogénocycloalkyle en C₃-C₈, un alcérzyle en C₂-C₈, un halogénoalcényle en C₂-C₈, un cycloalcényle en C₃-C₆, un halogénocycloalcényle en C₃-C₆, un alcinyle en C₂-C₈, un halogénoalcinyle en C₂-C₈ ou un phényle, naphtyle ou un hétérocycle de cinq ou six membres saturé, partiellement insaturé ou aromatique contenant un à quatre hétéroatomes du groupe O, N ou S,
R² signifie hydrogène ou un des groupes mentionnés en R¹,
R¹ et R² peuvent également former, avec l'atome d'azote auquel ils sont liés, un hétérocyclyle ou un hétéroaryle de cinq ou six membres, lequel est lié par N et peut contenir un à trois autres hétéroatomes du groupe 0, N et S comme élément de cycle et porter un ou plusieurs substituants du groupe des halogène, alkyle en C₁-C₆, halogénoalkyle en C₁-C₆, alcényle en C₂-C₆, halogénoalcényle en C₂-C₆, alcoxy en C₁-C₆, halogénoalcoxy en C₁-C₆, alcényloxy en C₃-C₆, halogénoalcényloxy en C₃-C₆, (exo)alkylène en C₁-C₆ et oxyalkylènoxy en C₁-C₃ ;
R¹ et/ou R² peuvent porter un à quatre groupes R^{a} identiques ou différents :
R^{a} signifie halogène, cyano, nitro, hydroxy, alkyle en C₁-C₆, halogénoalkyle en C₁-C₆, alkylcarbonyle en C₁-C₆, cycloalkyle en C₃-C₆, alcoxy en C₁-C₆, halogénoalcoxy en C₁-C₆, alcoxycarbonyle en C₁-C₆, alkylthio en C₁-C₆, alkylamino en C₁-C₆, dialkylamino en C₁-C₆, alcényle en C₂-C₈, halogénoalcényle en C₂-C₈, cycloalcényle en C₃-C₈, alcényloxy en C₂-C₆, halogénoalcényloxy en C₃-C₆, alcinyle en C₂-C₆, halogénoalcinyle en C₂-C₆, alcinyloxy en C₃-C₆, halogénoalcinyloxy en C₃-C₆, cycloalcoxy en C₃-C₆, cycloalcényloxy en C₃-C₆, oxyalkylènoxy en C₁-C₃, phényle, naphtyle, un hétérocycle de cinq à dix membres saturé, partiellement insaturé ou aromatique, contenant un à quatre hétéroatomes du groupe O, N ou S,
ces groupes aliphatiques, alicycliques ou aromatiques étant de leur côté partiellement ou complètement halogénés ou pouvant porter un à trois groupes R^{b} :
R^{b} signifie halogène, cyano, nitro, hydroxy, mercapto, amino, carboxyle, aminocarbonyle, aminothiocarbonyle, alkyle, halogénoalkyle, alcényle, alcényloxy, alcinyloxy, alcoxy, halogénoalcoxy, alkylthio, alkylamino, dialkylamino, formyle, alkylcarbonyle, alkylsulfonyle, alkylsulfoxyle, alcoxycarbonyle, alkylcarbonyloxy, alkylaminocarbonyle, dialkylaminocarbonyle, alkylaminothiocarbonyle, dialkylaminothiocarbonyle, les groupes alkyle dans ces radicaux contenant 1 à 6 atomes de carbone et les groupes alcényle ou alcinyle mentionnés dans ces radicaux contenant 2 à 8 atomes de carbone ;
et/ou un à trois des radicaux suivants :
cycloalkyle, cycloalcoxy, hétérocyclyle, hétérocyclyloxy, les systèmes cycliques contenant 3 à 10 éléments de cycle ; aryle, aryloxy, arylthio, aryl-alcoxy en C₁-C₆, aryl-alkyle en C₁-C₆, hétaryle, hétaryloxy, hétarylthio, les radicaux aryle contenant de préférence 6 à 10 éléments de cycle, les radicaux hétaryle contenant 5 ou 6 éléments de cycle, les systèmes cycliques pouvant être partiellement ou complètement halogénés ou être substitués par des groupes alkyle ou halogénoalkyle ;
L fluor, chlore ou méthyle,
X cyano, alkyle en C₁-C₄, alcoxy en C₁-C₄ ou halogénoalcoxy en C₁-C₂ ;
X ne signifiant pas méthyle, quand R¹ et R² signifient ensemble n-pentylène ou 3-méthyl-n-pentylène et L signifiant fluor, ou R¹ et R² signifient ensemble 3-méthyl-n-pentylène et L signifie chlore.

2. Composés de formule I selon la revendication 1, étant entendu que R¹ et R² ne peuvent signifier ensemble pipéridin-1-yle ou 4-alkyl-pipéridin-1-yle.

3. Composés de formule I selon la revendication 1, dans laquelle X signifie cyano, alcoxy en C₁-C₄ ou halogénoalcoxy en C₁-C₂.

4. Composés de formule I selon la revendication 1, dans laquelle X signifie alkyle en C₁-C₄.

5. Composés de formule I selon l'une des revendications 1 à 4, dans laquelle L signifie méthyle.

6. Composés de formule I selon l'une des revendications 1 à 5, dans laquelle R¹ signifie alkyle en C₁-C₈, halogénoalkyle en C₁-C₈, cycloalkyle en C₃-C₈, halogénocycloalkyle en C₃-C₈, alcényle en C₂-C₈, halogénoalcényle en C₂-C₈, cycloalcényle en C₃-C₆, halogénocycloalcényle en C₃-C₆, alcinyle en C₂-C₈, halogénoalcinyle en C₂-C₈ ou phényle, naphtyle ou un hétérocycle de cinq ou six membres saturé, partiellement insaturé ou aromatique contenant un à quatre hétéroatomes du groupe O, N ou S et R² signifie hydrogène ou alkyle en C₁-C₄, ou forment, avec l'atome d'azote auquel ils sont liés, un hétérocyclyle ou un hétéroaryle à cinq membres, lequel est lié par N et contient un à trois autres hétéroatomes du groupe O, N et S comme élément de cycle R¹ et R² pouvant être substitués selon la revendication 1.

7. Composés de formule I selon l'une des revendications 1 à 6, dans laquelle R² ne signifie pas hydrogène.

8. Composés de formule I selon la revendication 1, correspondant à la formule I.1 : 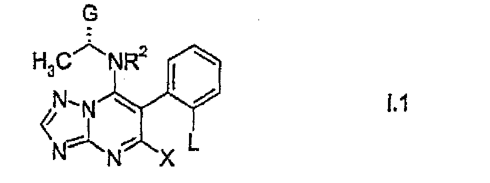 dans laquelle G signifie un alkyle en C₂-C₆, un alcoxyméthyle en C₁-C₄ ou un cycloalkyle C₃-C₆.

9. Composés de formule I selon la revendication 1, correspondant à la formule 1.2 : 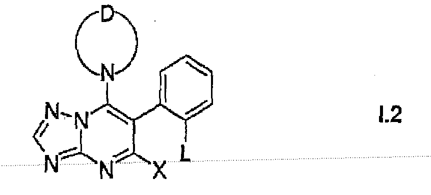 dans laquelle D, avec l'atome d'azote, forme un hétérocyclyle ou un hétéroaryle de cinq ou six membres, lequel est lié par N, et peut contenir comme élément de cycle un autre hétéroatome du groupe O, N et S et/ou peut porter un ou plusieurs substituants du groupe des halogène, alkyle en C₁-C₄, alcoxy en C₁-C₄ et halogénoalkyle en C₁-C₂.

10. Procédé de préparation des composés de formule I selon la revendication 1, dans laquelle X représente un cyano, un alkyle en C₁-C₄, un alcoxy en C₁-C₄ ou un halogénoalcoxy en C₁-C₂, par réaction de 5-aminotriazole de formule II 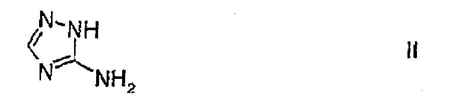 avec des phénylmalonates de formule III, 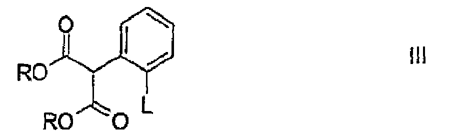 dans laquelle R représente un alkyle, pour obtenir des dihydroxytriazolopyrimidines de formule IV, 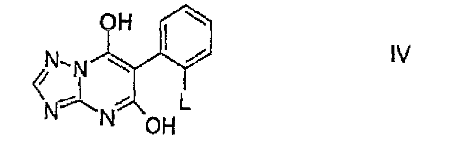 halogénation pour obtenir les composés dihalogénés de formule V, 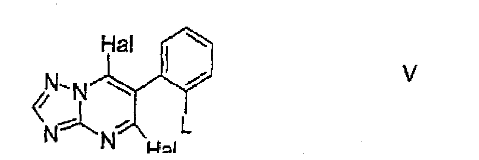 et réaction de V avec des amines de formule VI 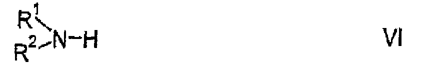 pour obtenir des composés de formule VII, 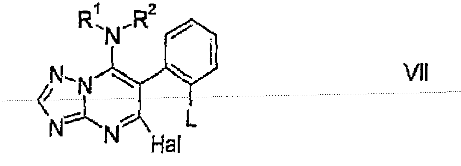 réaction des composés VII avec des composés de formule VIII,
M-X' VIII
qui, en fonction du groupe X' à introduire, représentent un cyanure inorganique, un alcoxylate ou un halogénoalcoxylate et dans laquelle M représente un cation d'ammonium, de tétraalkylammonium, de métal alcalin ou alcalino-terreux et, si on le souhaite, pour la préparation de composés de formule I selon la revendication 1, dans laquelle X représente alkyle, par réaction des composés VII avec des malonates de formule IX. 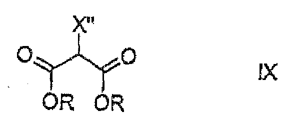 dans laquelle X" représente un hydrogène ou un alkyle en C₁-C₃ et R signifie un alkyle C₁-C₄, pour obtenir des composés de formule X 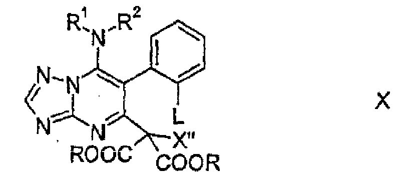 et décarboxylation pour obtenir des composés 1, dans lesquels X représente alkyle.

11. Procédé de préparation des composés de formule I selon la revendication 1, dans laquelle X représente un alkyle en C₁-C₄ ou un halogénoalkyle en C₁-C₄, par réaction de 5-aminotriazole de formule II selon la revendication 2 avec des cétoesters de formule IIIa, 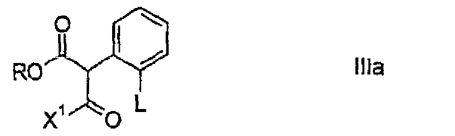 dans laquelle X¹ représente un alkyle en C₁-C₄ ou un halogénoalkyle en C₁-C₄ et R représente un alkyle en C₁-C₄, pour obtenir des 5-alkyl-7-hydroxy-6-phényltriazolopyrimidines de formule IVa 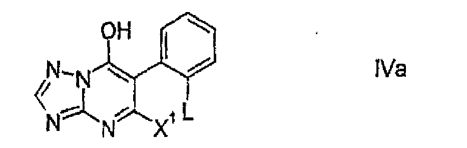 halogénation de IVa en 7-halogénotriazolopyrimidines de formule Va 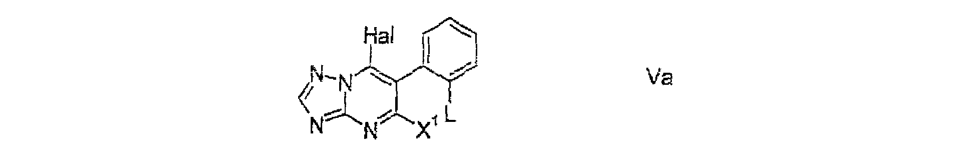 et réaction de Va avec des amines de formule VI selon la revendication 2 pour obtenir des composés I, dans lesquels X représente un alkyle en C₁-C₄ ou un halogénoalkyle en C₁-C₄.

12. Produit fongicide, contenant un support solide ou liquide ainsi qu'un composé de formule I selon la revendication 1.

13. Semences, contenant de 1 à 1000 g d'un composé de formule I selon la revendication 1 pour 100 kg.

14. Procédé de lutte contre les champignons nuisibles phytopathogènes, **caractérisé en ce qu'**on traite les champignons ou les matériaux, végétaux, sols ou semences à protéger des attaques de champignons avec une quantité efficace d'un composé de formule I selon la revendication 1.
